# EUROPEAN PATENT SPECIFICATION

(11) **EP 1 743 033 B1**
(45) Date of publication and mention of the grant of the patent: **19.10.2011**
(21) Application number: 05716551.6
(22) Date of filing: 07.04.2005
(51) Int. Cl.: C12Q 1/34, G01N 33/50

(54) **GM3 SYNTHASE AS A THERAPEUTIC TARGET IN MICROVASCULAR COMPLICATIONS OF DIABETES**
GM3-SYNTHASE ALS THERAPEUTISCHES ZIEL BEI MIKROVASKULÄREN KOMPLIKATIONEN DES DIABETES
GM3 SYNTHASE UTILISEE COMME CIBLE THERAPEUTIQUE DANS DES COMPLICATIONS MICROVASCULAIRES DU DIABETE

(30) Priority: 07.05.2004 FR 0404971; 17.12.2004 FR 0413530
(43) Date of publication of application: 17.01.2007
(73) Proprietor: Merck Patent GmbH, 64293 Darmstadt (DE)
(72) Inventor: ELBAWAB, Samer, F-75014 Paris (FR); MASSON, Elodie, F-69100 Villeurbanne (FR); RUGGIERO, Daniel, 60594 Frankfurt/Main (DE); WIERNSPERGER, Nicolas, F-69530 Orlienas (FR); LAGARDE, Michel, F-69150 Decines (FR); TRONCY, Lysiane, F-69870 Lamure-sur-Azergues (FR)
(86) International application number: PCT/EP2005/003647
(87) International publication number: WO 2005/108600

(56) References cited:
- AUDREY DALEME-NATALIZIO: "THESE : Etude des glycosphingolipides des cellules microvasculaires rétiniennes: effet d'un environnement diabétique; pages 158-199" 8 February 2002 (2002-02-08), INSTITUT NATIONAL DES SCIENCES APPLIQUEES DE LYON / UNIVERSITE LYON 1 , LYON , XP002312899 p.189, parargraphe 1.2 et p. 190-191, "discussion" page 168, line 1 - line 3 page 173, line 1 - line 4
- MASSON ELODIE ET AL: "a-series gangliosides mediate the effects of advanced glycation end products on pericyte and mesangial cell proliferation - A common mediator for retinal and renal microangiopathy?" DIABETES, vol. 54, no. 1, January 2005 (2005-01), pages 220-227, XP002346845 ISSN: 0012-1797
- STITT ALAN W: "The role of advanced glycation in the pathogenesis of diabetic retinopathy." EXPERIMENTAL AND MOLECULAR PATHOLOGY, vol. 75, no. 1, August 2003 (2003-08), pages 95-108, XP002312896 ISSN: 0014-4800
- WAUTIER J L ET AL: "Advanced glycation end products, their receptors and diabetic angiopathy" DIABETES AND METABOLISM, vol. 27, no. 5 Cahier 1, November 2001 (2001-11), pages 535-542, XP002312897 ISSN: 1262-3636
- NATALIZIO AUDREY ET AL: "Glycosphingolipid changes induced by advanced glycation end-products" BIOCHEMICAL AND BIOPHYSICAL RESEARCH COMMUNICATIONS, vol. 281, no. 1, 16 February 2001 (2001-02-16), pages 78-83, XP002312898 ISSN: 0006-291X

### SEQUENCE LISTING

<110> MERCK SANTE
   INSERM
<120> GM3 synthase as a therapeutic target in the microvascular complications of diabetes
<130> BFF 04P0578
<160> 5
<170> PatentIn version 3.1
<210> 1
   <211> 2362
   <212 > DNA
   <213> Homo sapiens
<220>
   <221> CDS
   <222> (278)..(1366)
   <223>
<400> 1
   ctgagcgggg gagcggcggc ccccagctga atgggcgcga gagcggcgct gggggcgggt      60
   gggggcgcgg ggtaccgggc tggcggccgg ccggcgcccc ctcattagta tgcggacgaa     120
   ggcggcgggc tgcgcggagc ggcgtcccct gcagccgcgg accgaggcag cggcggcacc     180
   tgccggccga gcaatgccaa gtgagtacac ctatgtgaaa ctgagaagtg attgctcgag     240
   gccttccctg caatggtaca cccgagctca aagcaag atg aga agg ccc agc ttg      295
                                           Met Arg Arg Pro Ser Leu
                                           1               5
   tta tta aaa gac atc ctc aaa tgt aca ttg ctt gtg ttt gga gtg tgg       343
   Leu Leu Lys Asp Ile Leu Lys Cys Thr Leu Leu Val Phe Gly Val Trp
               10                  15                  20
   atc ctt tat atc ctc aag tta aat tat act act gaa gaa tgt gac atg       391
   Ile Leu Tyr Ile Leu Lys Leu Asn Tyr Thr Thr Glu Glu Cys Asp Met
           25                  30                  35
   aaa aaa atg cat tat gtg gac cct gac cat gta aag aga gct cag aaa       439
   Lys Lys Met His Tyr Val Asp Pro Asp His Val Lys Arg Ala Gln Lys
       40                  45                  50
   tat gct cag caa gtc ttg cag aag gaa tgt cgt ccc aag ttt gcc aag       487
   Tyr Ala Gln Gln Val Leu Gln Lys Glu Cys Arg Pro Lys Phe Ala Lys
   55                  60                  65                  70
   aca tca atg gcg ctg tta ttt gag cac agg tat agc gtg gac tta ctc       535
   Thr Ser Met Ala Leu Leu Phe Glu His Arg Tyr Ser Val Asp Leu Leu
                   75                  80                  85
   cct ttt gtg cag aag gcc ccc aaa gac agt gaa gct gag tcc aag tac      583
   Pro Phe Val Gln Lys Ala Pro Lys Asp Ser Glu Ala Glu Ser Lys Tyr
               90                  95                  100
   gat cct cct ttt ggg ttc cgg aag ttc tcc agt aaa gtc cag acc ctc      631
   Asp Pro Pro Phe Gly Phe Arg Lys Phe Ser Ser Lys Val Gln Thr Leu
           105                 110                 115
   ttg gaa ctc ttg cca gag cac gac ctc cct gaa cac ttg aaa gcc aag      679
   Leu Glu Leu Leu Pro Glu His Asp Leu Pro Glu His Leu Lys Ala Lys
       120                 125                 130
   acc tgt cgg cgc tgt gtg gtt att gga agc gga gga ata ctg cac gga      727
   Thr Cys Arg Arg Cys Val Val Ile Gly Ser Gly Gly Ile Leu His Gly
   135                 140                 145                 150
   tta gaa ctg ggc cac acc ctg aac cag ttc gat gtt gtg ata agg tta      775
   Leu Glu Leu Gly His Thr Leu Asn Gln Phe Asp Val Val Ile Arg Leu
                   155                 160                 165
   aac agt gca cca gtt gag gga tat tca gaa cat gtt gga aat aaa act      823
   Asn Ser Ala Pro Val Glu Gly Tyr Ser Glu His Val Gly Asn Lys Thr
               170                 175                 180
   act ata agg atg act tat cca gag ggc gca cca ctg tct gac ctt gaa      871
   Thr Ile Arg Met Thr Tyr Pro Glu Gly Ala Pro Leu Ser Asp Leu Glu
           185                 190                 195
   tat tat tcc aat gac tta ttt gtt gct gtt tta ttt aag agt gtt gat      919
   Tyr Tyr Ser Asn Asp Leu Phe Val Ala Val Leu Phe Lys Ser Val Asp
       200                 205                 210
   ttc aac tgg ctt caa gca atg gta aaa aag gaa acc ctg cca ttc tgg      967
   Phe Asn Trp Leu Gln Ala Met Val Lys Lys Glu Thr Leu Pro Phe Trp
   215                 220                 225                 230
   gta cga ctc ttc ttt tgg aag cag gtg gca gaa aaa atc cca ctg cag     1015
   Val Arg Leu Phe Phe Trp Lys Gln Val Ala Glu Lys Ile Pro Leu Gln
                   235                 240                 245
   cca aaa cat ttc agg att ttg aat cca gtt atc atc aaa gag act gcc     1063
   Pro Lys His Phe Arg Ile Leu Asn Pro Val Ile Ile Lys Glu Thr Ala
               250                 255                 260
   ttt gac atc ctt cag tac tca gag cct cag tca agg ttc tgg ggc cga     1111
   Phe Asp Ile Leu Gln Tyr Ser Glu Pro Gln Ser Arg Phe Trp Gly Arg
           265                 270                 275
   gat aag aac gtc ccc aca atc ggt gtc att gcc gtt gtc tta gcc aca     1159
   Asp Lys Asn Val Pro Thr Ile Gly Val Ile Ala Val Val Leu Ala Thr
       280                 285                 290
   cat ctg tgc gat gaa gtc agt ttg gcg ggt ttt gga tat gac ctc aat     1207
   His Leu Cys Asp Glu Val Ser Leu Ala Gly Phe Gly Tyr Asp Leu Asn
   295                 300                 305                 310
   caa ccc aga aca cct ttg cac tac ttc gac agt caa tgc atg gct gct     1255
   Gln Pro Arg Thr Pro Leu His Tyr Phe Asp Ser Gln Cys Met Ala Ala
                   315                 320                 325
   atg aac ttt cag acc atg cat aat gtg aca acg gaa acc aag ttc ctc      1303
   Met Asn Phe Gln Thr Met His Asn Val Thr Thr Glu Thr Lys Phe Leu
                   330                 335                 340
   tta aag ctg gtc aaa gag gga gtg gtg aaa gat ctc agt gga ggc att      1351
   Leu Lys Leu Val Lys Glu Gly Val Val Lys Asp Leu Ser Gly Gly Ile
           345                 350                 355
   gat cgt gaa ttt tga acacagaaaa cctcagttga aaatgcaact ctaactctga      1406
   Asp Arg Glu Phe
       360
   gagctgtttt tgacagcctt cttgatgtat ttctccatcc tgcagatact ttgaagtgca   1466
   gctcatgttt ttaactttta atttaaaaac acaaaaaaaa ttttagctct tcccactttt   1526
   tttttcctat ttatttgagg tcagtgtttg tttttgcaca ccattttgta aatgaaactt   1586
   aagaattgaa ttggaaagac ttctcaaaga gaattgtatg taacgatgtt gtattgattt   1646
   ttaagaaagt aatttaattt gtaaaacttc tgctcgttta cactgcacat tgaatacagg   1706
   taactaattg gaaggagagg ggaggtcact cttttgatgg tggccctgaa cctcattctg   1766
   gttccctgct gcgctgcttg gtgtgaccca cggaggatcc actcccagga tgacgtgctc   1826
   cgtagctctg ctgctgatac tgggtctgcg atgcagcggc gtgaggcctg ggctggttgg   1886
   agaaggtcac aacccttctc tgttggtctg ccttctgctg aaagactcga gaaccaacca   1946
   gggaagctgt cctggaggtc cctggtcgga gagggacata gaatctgtga cctctgacaa   2006
   ctgtgaagcc accctgggct acagaaacca cagtcttccc agcaattatt acaattcttg   2066
   aattccttgg ggatttttta ctgccctttc aaagcactta agtgttagat ctaacgtgtt   2126
   ccagtgtctg tctgaggtga cttaaaaaat cagaacaaaa cttctattat ccagagtcat   2186
   gggagagtac accctttcca ggaataatgt tttgggaaac actgaaatga aatcttccca   2246
   gtattataaa ttgtgtattt aaaaaaaaga aacttttctg aatgcctacc tggcggtgta   2306
   taccaggcag tgtgccagtt taaaaagatg aaaaagaata aaaacttttg aggaac       2362
<210> 2
<211> 362
<212> PRT
   <213> Homo sapiens
<400> 2
   Met Arg Arg Pro Ser Leu Leu Leu Lys Asp Ile Leu Lys Cys Thr Leu
   1               5                   10                  15
   Leu Val Phe Gly Val Trp Ile Leu Tyr Ile Leu Lys Leu Asn Tyr Thr
               20                  25                  30
   Thr Glu Glu Cys Asp Met Lys Lys Met His Tyr Val Asp Pro Asp His
   35 40 45
   Val Lys Arg Ala Gln Lys Tyr Ala Gln Gln Val Leu Gln Lys Glu Cys
   50 55 60
   Arg Pro Lys Phe Ala Lys Thr Ser Met Ala Leu Leu Phe Glu His Arg
   65 70 75 80
   Tyr Ser Val Asp Leu Leu Pro Phe Val Gln Lys Ala Pro Lys Asp Ser
   85 90 95
   Glu Ala Glu Ser Lys Tyr Asp Pro Pro Phe Gly Phe Arg Lys Phe Ser
   100 105 110
   Ser Lys Val Gln Thr Leu Leu Glu Leu Leu Pro Glu His Asp Leu Pro
   115 120 125
   Glu His Leu Lys Ala Lys Thr Cys Arg Arg Cys Val Val Ile Gly Ser
   130 135 140
   Gly Gly Ile Leu His Gly Leu Glu Leu Gly His Thr Leu Asn Gln Phe
   145 150 155 160
   Asp Val Val Ile Arg Leu Asn Ser Ala Pro Val Glu Gly Tyr Ser Glu
   165 170 175
   His Val Gly Asn Lys Thr Thr Ile Arg Met Thr Tyr Pro Glu Gly Ala
   180 185 190
   Pro Leu Ser Asp Leu Glu Tyr Tyr Ser Asn Asp Leu Phe Val Ala Val
   195 200 205
   Leu Phe Lys Ser Val Asp Phe Asn Trp Leu Gln Ala Met Val Lys Lys
   210 215 220
   Glu Thr Leu Pro Phe Trp Val Arg Leu Phe Phe Trp Lys Gln Val Ala
   225 230 235 240
   Glu Lys Ile Pro Leu Gln Pro Lys His Phe Arg Ile Leu Asn Pro Val
   245 250 255
   Ile Ile Lys Glu Thr Ala Phe Asp Ile Leu Gln Tyr Ser Glu Pro Gln
   260 265 270
   Ser Arg Phe Trp Gly Arg Asp Lys Asn Val Pro Thr Ile Gly Val Ile
   275 280 285
   Ala Val Val Leu Ala Thr His Leu Cys Asp Glu Val Ser Leu Ala Gly
   290 295 300
   Phe Gly Tyr Asp Leu Asn Gln Pro Arg Thr Pro Leu His Tyr Phe Asp
   305 310 315 320
   Ser Gln Cys Met Ala Ala Met Asn Phe Gln Thr Met His Asn Val Thr
   325 330 335
   Thr Glu Thr Lys Phe Leu Leu Lys Leu Val Lys Glu Gly Val Val Lys
   340 345 350
   Asp Leu Ser Gly Gly Ile Asp Arg Glu Phe
   355 360
<210> 3
   <211> 2221
   <212> DNA
   <213> Mus musculus
<220>
   <221> CDS
   <222> (151)..(1314)
   <223>
<400> 3
   tctttgcgat accccaggcc cagcggctcc tccccagccc tgcgacgccg gacgcgcctg      60
   ctaggggaca cgggcggagg gtcgcggccc ctggctgcct acatgggcgc ccccggcgag     120
   ctgcgcaggt gtggacgcgg cgctgcggca atg cca agt gag ttc acc tct gca      174
                                    Met Pro Ser Glu Phe Thr Ser Ala
                                    1               5
   aag ctg aga agt gat tgc tca agg acc tcc ctg caa tgg tac acc cga       222
   Lys Leu Arg Ser Asp Cys Ser Arg Thr Ser Leu Gln Trp Tyr Thr Arg
       10                  15                  20
   acc cag cac aag atg aga aga ccc agc ttg tta ata aaa gac atc tgc       270
   Thr Gln His Lys Met Arg Arg Pro Ser Leu Leu Ile Lys Asp Ile Cys
   25                  30                  35                  40
   aag tgc acg ttg gtt gca ttt gga gtc tgg ctc ctg tac atc ctc att       318
   Lys Cys Thr Leu Val Ala Phe Gly Val Trp Leu Leu Tyr Ile Leu Ile
                   45                  50                  55
   ttg aat tac acc gct gaa gaa tgt gac atg aaa aga atg cac tat gtg        366
   Leu Asn Tyr Thr Ala Glu Glu Cys Asp Met Lys Arg Met His Tyr Val
               60                  65                  70
   gac cct gac cgg ata aag aga gct cag agc tat gct cag gaa gtc ttg        414
   Asp Pro Asp Arg Ile Lys Arg Ala Gln Ser Tyr Ala Gln Glu Val Leu
           75                  80                  85
   cag aag gaa tgt cgg ccc agg tac gcg aag acg gct atg gct ctg tta        462
   Gln Lys Glu Cys Arg Pro Arg Tyr Ala Lys Thr Ala Met Ala Leu Leu
       90                  95                  100
   ttt gag gac agg tac agc atc aac ttg gag cct ttt gtg cag aag gtc        510
   Phe Glu Asp Arg Tyr Ser Ile Asn Leu Glu Pro Phe Val Gln Lys Val
   105                 110                 115                 120
   ccc acg gcc agt gaa gct gag ctc aag tat gac ccg cct ttt gga ttc        558
   Pro Thr Ala Ser Glu Ala Glu Leu Lys Tyr Asp Pro Pro Phe Gly Phe
                   125                 130                 135
   cgg aag ttc tcc agt aaa gtc cag agc ctc ttg gat atg ctg ccc gaa        606
   Arg Lys Phe Ser Ser Lys Val Gln Ser Leu Leu Asp Met Leu Pro Glu
               140                 145                 150
   cat gac ttt tct gaa cac ttg aga gcc aag gcc tgc aag cgc tgt gtg        654
   His Asp Phe Ser Glu His Leu Arg Ala Lys Ala Cys Lys Arg Cys Val
           155                 160                 165
   gtt gtt ggg aac ggg ggc atc ctg cac gga cta gag ctg ggt cac gcc        702
   Val Val Gly Asn Gly Gly Ile Leu His Gly Leu Glu Leu Gly His Ala
       170                 175                 180
   ctc aac cag ttc gat gtg gta ata agg ttg aac agt gcg cca gtt gag        750
   Leu Asn Gln Phe Asp Val Val Ile Arg Leu Asn Ser Ala Pro Val Glu
   185                 190                 195                 200
   ggt tac tct gaa cac gtt ggg aat aaa act act ata agg atg act tac        798
   Gly Tyr Ser Glu His Val Gly Asn Lys Thr Thr Ile Arg Met Thr Tyr
                   205                 210                 215
   cca gag ggt gcg cca ctg tcg gac gtt gaa tac tac gcc aat gat ttg        846
   Pro Glu Gly Ala Pro Leu Ser Asp Val Glu Tyr Tyr Ala Asn Asp Leu
               220                 225                 230
   ttc gtt act gtt tta ttt aag agt gtt gat ttc aag tgg ctt caa gca        894
   Phe Val Thr Val Leu Phe Lys Ser Val Asp Phe Lys Trp Leu Gln Ala
           235                 240                 245
   atg gta aaa aat gaa agc ctg ccc ttt tgg gtt cgc ctc ttc ttt tgg        942
   Met Val Lys Asn Glu Ser Leu Pro Phe Trp Val Arg Leu Phe Phe Trp
       250                 255                 260
   aag caa gtg gca gaa aaa gtc cca ctc cag cca aag cac ttc agg att        990
   Lys Gln Val Ala Glu Lys Val Pro Leu Gln Pro Lys His Phe Arg Ile
   265                 270                 275                 280
   ttg aac cca gtt atc atc aaa gaa act gcc ttc gac atc ctt cag tac       1038
   Leu Asn Pro Val Ile Ile Lys Glu Thr Ala Phe Asp Ile Leu Gln Tyr
                   285                 290                 295
   tca gag cct cag tca aga ttc tgg ggc cat gat aag aac atc ccc acg     1086
   Ser Glu Pro Gln Ser Arg Phe Trp Gly His Asp Lys Asn Ile Pro Thr
               300                 305                 310
   atc ggc gtc att gcc gtt gtc ttg gct aca cat ctg tgt gat gaa gtc     1134
   Ile Gly Val Ile Ala Val Val Leu Ala Thr His Leu Cys Asp Glu Val
           315                 320                 325
   agc ctg gca ggc ttt ggc tac gac ctc agt caa ccc agg acc cct ctg     1182
   Ser Leu Ala Gly Phe Gly Tyr Asp Leu Ser Gln Pro Arg Thr Pro Leu
       330                 335                 340
   cac tac ttt gac agt cag tgc atg ggc gcc atg cac tgg cag gtc atg     1230
   His Tyr Phe Asp Ser Gln Cys Met Gly Ala Met His Trp Gln Val Met
   345                 350                 355                 360
   cac aat gtg acc aca gag acc aag ttc ctc ctg aag ctc ctc aag gag     1278
   His Asn Val Thr Thr Glu Thr Lys Phe Leu Leu Lys Leu Leu Lys Glu
                   365                 370                 375
   ggc gtg gtg gag gac ctc agc ggc ggc atc cac tga gaactcggaa          1324
   Gly Val Val Glu Asp Leu Ser Gly Gly Ile His
               380                 385
   cacggcaaac ctcacccagc accgcagctg agagcgtggt gagcagcctc cacagggact   1384
   tcaccctgca gctgcttcga tgtgcagcta gtgttttcaa actccacatt ttttttaaaa    1444
   aaggaaaaga aagaacaaca gcaacaacaa aagctctgct ctgtgcacct cttcgtccta   1504
   tttatttgaa gtcagtgttg gattttgcac agttttgtaa gttaatctta agaatgggat   1564
   tggaaggact tttcaaagag aattgtatag tttattgttt tttaaggaag taatttaatt   1624
   tgcagaaact gtacacacgt actctgctca ggtgttgagg tgggaggaga ggggcttctg   1684
   gcccctggat gatggctgtg atgcccgata ctggggtctg ctgctctgtt tggtagaact   1744
   gatggcagag aaacttcctg cctccaggat aaagggctta ctcatcacct ctggcagctg   1804
   ctagacaagt tcataacccc tttctgctag tccatctgcc agctggctcg caggactcag   1864
   gcagggcagc tgtcccggag gctgctggtt ggtgagccac tgtcagctga gcgccgtgat   1924
   gttgccccag ggtggaagaa gccacacttc ctacactgtc agggcacttt taaacttctg   1984
   gaggggtgtg tgtgtgtgtg tgtgtgtgtg tgtgtgtgtg tgtgtgtgtg tgtgtgttca   2044
   ttctgccctt ccaaatcatc taagtgttat ttaaggcact ctgctgtttg tatgagatgg   2104
   ctcatagata ttatgacaaa gcctttgtta tccaggccat gggaagagga aaaagaaaag   2164
   aaagagagaa aagaataaaa gcttttgagg agcccctgtg atttcctgaa aaaaaaa      2221
<210> 4
   <211> 387
   <212> PRT
   <213> Mus musculus
<400> 4
   Met Pro Ser Glu Phe Thr Ser Ala Lys Leu Arg Ser Asp Cys Ser Arg
   1               5                   10                  15
   Thr Ser Leu Gln Trp Tyr Thr Arg Thr Gln His Lys Met Arg Arg Pro
               20                  25                  30
   Ser Leu Leu Ile Lys Asp Ile Cys Lys Cys Thr Leu Val Ala Phe Gly
           35                  40                  45
   Val Trp Leu Leu Tyr Ile Leu Ile Leu Asn Tyr Thr Ala Glu Glu Cys
       50                  55                  60
   Asp Met Lys Arg Met His Tyr Val Asp Pro Asp Arg Ile Lys Arg Ala
   65                  70                  75                  80
   Gln Ser Tyr Ala Gln Glu Val Leu Gln Lys Glu Cys Arg Pro Arg Tyr
                   85                  90                  95
   Ala Lys Thr Ala Met Ala Leu Leu Phe Glu Asp Arg Tyr Ser Ile Asn
               100                 105                 110
   Leu Glu Pro Phe Val Gln Lys Val Pro Thr Ala Ser Glu Ala Glu Leu
           115                 120                 125
   Lys Tyr Asp Pro Pro Phe Gly Phe Arg Lys Phe Ser Ser Lys Val Gln
       130                 135                 140
   Ser Leu Leu Asp Met Leu Pro Glu His Asp Phe Ser Glu His Leu Arg
   145                 150                 155                 160
   Ala Lys Ala Cys Lys Arg Cys Val Val Val Gly Asn Gly Gly Ile Leu
                   165                 170                 175
   His Gly Leu Glu Leu Gly His Ala Leu Asn Gln Phe Asp Val Val Ile
               180                 185                 190
   Arg Leu Asn Ser Ala Pro Val Glu Gly Tyr Ser Glu His Val Gly Asn
           195                 200                 205
   Lys Thr Thr Ile Arg Met Thr Tyr Pro Glu Gly Ala Pro Leu Ser Asp
       210                 215                 220
   Val Glu Tyr Tyr Ala Asn Asp Leu Phe Val Thr Val Leu Phe Lys Ser
   225                 230                 235                 240
   Val Asp Phe Lys Trp Leu Gln Ala Met Val Lys Asn Glu Ser Leu Pro
                   245                 250                 255
   Phe Trp Val Arg Leu Phe Phe Trp Lys Gln Val Ala Glu Lys Val Pro
               260                 265                 270
   Leu Gln Pro Lys His Phe Arg Ile Leu Asn Pro Val Ile Ile Lys Glu
           275                 280                 285
   Thr Ala Phe Asp Ile Leu Gln Tyr Ser Glu Pro Gln Ser Arg Phe Trp
       290                 295                 300
   Gly His Asp Lys Asn Ile Pro Thr Ile Gly Val Ile Ala Val Val Leu
   305                 310                 315                 320
   Ala Thr His Leu Cys Asp Glu Val Ser Leu Ala Gly Phe Gly Tyr Asp
                   325                 330                 335
   Leu Ser Gln Pro Arg Thr Pro Leu His Tyr Phe Asp Ser Gln Cys Met
               340                 345                 350
   Gly Ala Met His Trp Gln Val Met His Asn Val Thr Thr Glu Thr Lys
           355                 360                 365
   Phe Leu Leu Lys Leu Leu Lys Glu Gly Val Val Glu Asp Leu Ser Gly
       370                 375                 380
   Gly Ile His
   385
<210> 5
   <211> 21
   <212> RNA
   <213> Artificial Sequence
<220>
   <223> Interfering RNA
<220>
   <221> misc_feature
   <222> (20)..(21)
   <223> n = t 5-methyluridine
<400> 5
   ggguuauucu gaacauguun n 21

## Description

The invention relates to the use of GM3 synthase inhibitors for treating microvascular complications of diabetes, and to a method of screening inhibitors of this enzyme, and wherein the microvascular complication of diabetis is diabetic nephropathy.

Microangiopathy is a chronic complication of diabetes which is characterised by structural and functional changes to the microvessels. The retina and the kidney are the two main targets of the pathological condition, leading to diabetic retinopathy and diabetic nephropathy.

Diabetic retinopathy is the second cause of blindness in developed countries. After about twenty years of the disease, almost all patients with type 1 diabetes and more than 60% of patients with type 2 diabetes suffer from this microvascular complication (Fong et al., 2003). The capillaries undergo progressive structural changes, such as a thickening of the basal membrane and the specific loss of pericytes, and subsequent modifications of the proliferation and function of the endothelial cells. These changes, combined with ischaemia, damage the microvessel wall and promote an excessive capillary permeability, leading to oedemas, microaneurisms and haemorrhages that threaten eyesight (Forrester et al., 1997).

Nephropathy affects 50 to 60% of patients with a 20- to 30-year history of diabetes. It is considered to be a major cause of mortality in these patients (Krolewski et al., 1997). One of the main characteristics of the pathological condition is glomerular enlargement, which is a consequence of thickening of the basal membrane and expansion of the mesangium due to hypertrophy of the mesangial cells in arrested growth and due to the accumulation of proteins of the extracellular matrix. These events, combined with haemodynamic deficiencies, induce glomerular sclerosis, glomerular filtration or a changed level of glomerular filtration and microalbuminuria, leading to severe renal insufficiency (Wolf et al., 2000).

The risk of a diabetic subject developing nephropathy is generally evaluated by checking the microalbuminuria. Preventive or therapeutic means currently used for delaying the appearance and/or progression of diabetic nephropathy include the monitoring of glycaemia, the administration of antihypertensives, especially angiotensin converting enzyme inhibitors, the adoption of a low-protein diet or the administration of hypolipidaemics such as statins (Rippin et al., 2004).

Given their impact in terms of public health and economics, the identification of novel ways of preventing and treating microvascular complications of diabetes constitutes a major therapeutic challenge.

Although the cellular and molecular basis of the pathogenesis of diabetic retinopathy and diabetic nephropathy is not completely understood, the regulation of cell proliferation and the cell-cell and cell-matrix interactions seem to play an important role. A number of biochemical hypotheses have been proposed to explain the mechanisms involved in the development of microvascular complications of diabetes, especially the formation of advanced glycation end products (AGE) (Singh et al., 2001).

Reducing sugars such as glucose react non-enzymatically with the amino groups of proteins, lipids and nucleic acids via a series of reactions that form Schiff bases and Amadori products, ultimately producing AGE. Glycation, which is dependent on glucose concentration, is increased in diabetes. It occurs preferentially with long-lived proteins exposed to the blood glucose, such as the proteins of the extracellular matrix or the circulating proteins, thereby modifying their structure and their function.

In addition, AGE can bind membrane receptors to induce cellular responses via the generation of oxidative stress (Lal et al., 2002; Schmidt et al., 1994), the activation of nuclear factor κB (Singh et al., 2001; Schmidt et al., 1994) and the expression of different genes such as proinflammatory cytokines or adhesion molecules (Hofmann et al., 1999; Schmidt et al., 1995).

All these modifications have important biological effects which can explain many of the changes observed in microvascular complications of diabetes, especially the increase in vascular permeability, the increase in the production and rigidity of the extracellular matrix, and the change in cell-matrix interactions and cell growth (Stitt et al., 2003). In fact, many *in vivo* and *in vitro* studies have suggested that AGE are involved in the development of the retinopathy and nephropathy associated with diabetes (Stitt et al., 2003; Wautier et al., 2001).

Gangliosides are glycosphingolipids that are concentrated in microdomains of the plasmic membrane and characterised by the presence of sialic acid in their structure. Successive sialylations of lactosylceramide produce monosialoganglioside (GM3), disialoganglioside (GD3) and trisialoganglioside (GT3). These gangliosides are then converted by the sequential action of glycosyltransferases and sialyltransferases to more complex gangliosides, forming the a, b and c series, respectively (Van Echten et al., 1993). Gangliosides are known to play a major role in cell-cell and cell-matrix recognition by interaction with adhesion receptors such as integrins or matrix proteins (collagen and fibronectin), or with other glycosphingolipids. Also, gangliosides, particularly those of the a series, have been implicated in the regulation of cell proliferation via modulation of the activity of different growth factors (Hakomori et al., 1990).

It has been reported that AGE induce modifications to the metabolism of glycosphingolipids in pericytes and endothelial cells of retinal microvessels (Natalizio et al., 2001). These changes are accompanied especially by an increase in GM3 synthase activation (A. Daleme-Natalizio, doctoral thesis at the Institut National des Sciences Appliquées de Lyon, 8 February 2002).

The inventors have now shown that gangliosides are involved in the AGE-mediated effects that lead to the pathological conditions DR and DN. The inventors have thus demonstrated that the inhibition by AGE of the proliferation of retinal pericytes and renal mesangial cells - the two cell types involved in diabetic retinopathy and diabetic nephropathy, respectively - is at least partly based on the increase in GM3 synthase activity and the accumulation of series a gangliosides. An increase in GM3 synthase activity has moreover been observed in a diabetic mouse model to which AGE have been given. These results identify GM3 synthase and series a gangliosides as targets for treating microvascular complications of diabetes.

### Definitions

The expression "microvascular complication of diabetes" denotes a chronic complication of type I or II diabetes that is characterised by structural and functional changes in the microvessels. These complications mainly include diabetic retinopathy, diabetic nephropathy and diabetic neuropathy. Peripheral neuropathy affects the nerves of the body extremities, loss of sensation in the feet being one of its most widespread forms. Discomfort and pain (paraesthesia and hyperaesthesia) are also very common and debilitating symptoms. This neuropathy can cause foot ulcers and serious tissue damage that may necessitate amputation.

Within the framework of the present patent application, the expression "GM3 synthase" denotes the enzyme lactosylceramide α2,3-sialyltransferase (EC 2.4.99.9), which catalyses the transfer of a sialic acid residue from a sialic acid donor to a 3-hydroxyl group of a galactose residue of a sialic acid acceptor. Preferentially, the sialic acid donor is CMP-N-acetylneuramic acid and the sialic acid acceptor is a galactose residue of a glycolipid such as lactosylceramide (LacCer). The catalysed reaction may be CMP-N-acetylneuraminate + β-D-galactosyl-1,4-β-D-glucosylceramide = Cmp + α-N-acetylneuraminyl-2,3-β-D-galactosyl-1,4-β-D-glucosylceramide. Preferably, the GM3 synthase according to the invention is a human GM3 synthase or a GM3 synthase of a non-human mammal such as a rodent (e.g. rat or mouse), a feline, a canine, a primate (monkey), etc. For example, the genes coding for human and murine GM3 synthase have been respectively deposited in the Genbank database under the accession numbers NM_003896 (SEQ ID No: 1) and NM_011375 (SEQ ID No: 3). The corresponding amino acid sequences are described in the sequences SEQ ID No: 2 and SEQ ID No: 4, respectively.

Within the framework of the present patent application, a "GM3 synthase inhibitor" denotes a compound which: (i) inhibits the activity and/or expression of GM3 synthase in vitro and/or in vivo; and/or (ii) blocks the transfer of a sialic acid residue from a sialic acid donor to a 3-hydroxyl group of a galactose residue of a sialic acid acceptor, especially to form ganglioside GM3; and/or (iii) blocks the intracellular synthesis of ganglioside GM3. The inhibition or blocking can be partial or total.

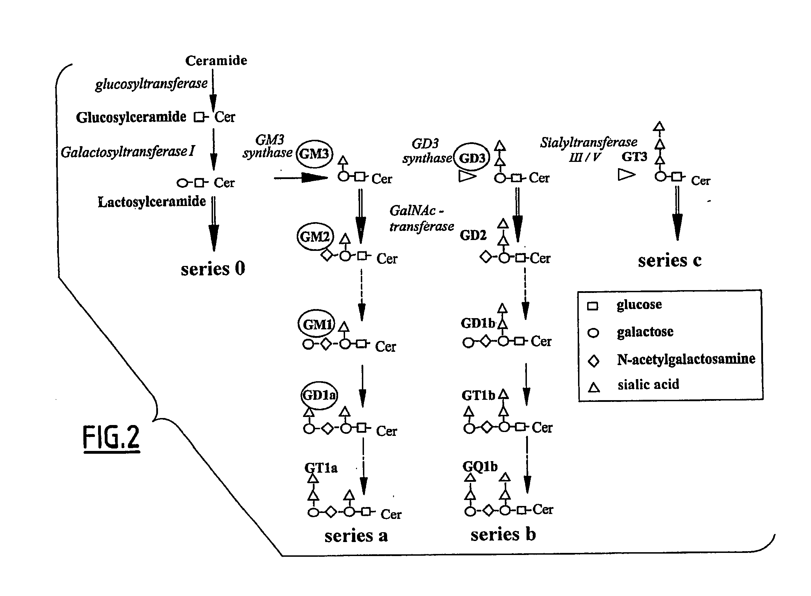

"Ganglioside" is understood as meaning a glycosphingolipid comprising one or more sialic acid residues. More specifically, "series a gangliosides" denote gangliosides carrying only one sialic acid residue on the galactose of the lactosylceramide. Series a gangliosides include the compounds GM3 (α-N-acetylneuraminyl-2,3-β-D-galactosyl-1,4-β-D-glucosylceramide), GM2, GM1, GD1a and GT1a (cf. Figure 2).

### Therapeutic application

The inventors have demonstrated that the advanced glycation end products (AGE) involved in the development of microvascular complications of diabetes mediate their effects via an increase in the activity of the GM3 synthase enzyme.

The invention therefore proposes a method of treating a microvascular complication of diabetes wherein an inhibitor of the expression or activity of the GM3 synthase gene is administered to the patient, and wherein the microvascular complication of diabetes is diabetic nephropathy.

The invention further relates to the use of an inhibitor of the expression or activity of the GM3 synthase gene for the manufacture of a drug intended for the treatment of a microvascular complication of diabetes, and wherein the microvascular complication of diabetes is diabetic nephropathy.

Within the framework of the invention, the expression "treatment" denotes the preventive or curative treatment of a disease, i.e. the act of reversing, slowing down or inhibiting the progression, or preventing the development, of a disease or of one or more symptoms attached to this disease.

The expression "patient" denotes a human or a non-human mammal, such as a mouse, rat, dog, cat, pig or monkey, that is affected or liable to be affected by a microvascular complication of diabetes. Preferably, a patient in terms of the invention is a subject in which diabetes has been detected.

Preferably, the inhibitor is a specific inhibitor of the expression or activity of the GM3 synthase gene, i.e. an inhibitor substantially devoid of an effect on genes or proteins other than GM3 synthase.

In a first embodiment, the method or use according to the invention employs an inhibitor of the expression of the GM3 synthase gene and/or protein. Such an inhibitor can inhibit or repress the transcription of the gene and/or the translation of the transcript messenger (mRNA). Those skilled in the art are capable of choosing the most suitable strategy for this purpose.

An antisense strategy can be used to inhibit GM3 synthase expression. This approach can utilise e.g. antisense nucleic acids or ribozymes which block the transcription of a specific mRNA, either by masking the mRNA with an antisense nucleic acid, or by cleaving the mRNA with a ribozyme. In the context of the present invention, "antisense" broadly includes RNA-RNA interactions, RNA-DNA interactions, ribozymes, interfering RNAs, aptamers, and inhibition mediated by RNAseH. An antisense therapy generally employs a vector, such as a viral vector, which carries the antisense sequence, the inhibition then being generally stable since the vector will integrate into the genome. It is also possible to use antisense oligonucleotides, which bring about a transitory inhibition of expression. A general presentation of the antisense technique can be found in "Antisense DNA and RNA" (Cold Spring Harbor Laboratory, D. Melton, ed., 1988).

Preferably, the inhibitor of the expression of the GM3 synthase gene and/or protein is therefore selected from the group comprising antisense nucleic acids, ribozymes, interfering RNAs and aptamers.

An "antisense nucleic acid" or an "antisense oligonucleotide" is a single-stranded nucleic acid molecule which, when it hybridises under cytoplasmic conditions with a complementary DNA or RNA molecule, inhibits the function of the latter. Antisense nucleic acids can be encoded by a recombinant gene for expression in a cell (cf., for example, US patents no. 5814500 and 5811234), or they can be prepared by synthesis (cf., for example, US patent no. 5780607). Antisense nucleic acids of GM3 synthase can be designed to hybridise specifically with a homologous sequence coding for a GM3 synthase, e.g. to hybridise specifically with the human GM3 synthase sequence shown in SEQ ID No: 1 or the murine GM3 synthase sequence shown in SEQ ID No: 3.

A "sequence capable of hybridising specifically with a nucleic acid sequence" denotes a sequence that hybridises with a reference nucleic acid sequence under highly stringent conditions (Sambrook et al., 1989). The parameters defining the stringency conditions depend on the temperature at which 50% of the matching strands separate (Tm), and on the ionic strength. For sequences comprising more than 30 bases, Tm is defined by the following equation: Tm = 81.5 + 0.41 (%G+C) + 16.6log(cation concentration) - 0.63(%formamide) - (600/number of bases) (Sambrook et al., 1989). For sequences shorter than 30 bases, Tm is defined by the following equation: Tm = 4(G+C) + 2(A+T). Under appropriate stringency conditions where the non-specific sequences do not hybridise, the hybridisation temperature can preferably be 5 to 10°C below Tm and the hybridisation buffers used are preferably solutions of high ionic strength, such as a 6x SSC solution. For example, highly stringent hybridisation conditions correspond to Tm and to ionic conditions such as those obtained with a solution containing 50% formamide and 5x or 6x SCC (0.15 M NaCl, 0.015 M sodium citrate).

The antisense nucleic acid according to the invention can be used as such, for example after injection into the human or animal, to induce protection or treat a microvascular complication of diabetes. In particular, they can be injected in the form of naked DNA according to the technique described in international patent application WO 90/11092. They can also be administered in the form of a complex with e.g. dextran-DEAE (Pagano et al., 1967), nuclear proteins (Kaneda et al., 1989) or lipids (Felgner et al., 1987), in the form of liposomes (Fraley et al., 1980) or by other similar methods.

Preferably, the nucleic acid sequences form part of a vector. The use of a vector makes it possible to improve the administration of the nucleic acid to the cells to be treated, and also improves the stability in these cells, affording a prolonged therapeutic effect.

The term "vector" denotes the vehicle via which a DNA or RNA sequence can be introduced into a host cell so as to transform the host and obtain the expression (i.e. the transcription and translation) of the sequence which has been introduced. Vectors include plasmids, phages, viruses, etc.

"Ribozymes" are RNA molecules that have the capacity specifically to cleave other single-stranded RNA molecules in a manner fairly analogous to DNA restriction endonucleases. Ribozymes were discovered by demonstrating that certain mRNAs have the capacity to cleave their own introns. By modifying the nucleotide sequence of these ribozymes, it is possible to produce molecules that recognise specific nucleotide sequences in RNA molecules and cleave them (Cech, 1989). Because of this specificity, only mRNAs that have a particular sequence are inactivated.

Reversible inhibition of GM3 synthase transcription can also be achieved using interfering RNAs. The technique of RNA interference (RNAi) prevents the expression of genes by using small RNA molecules such as "small interfering RNAs" (siRNAs). This technique benefits from the fact that RNA interference is a natural biological mechanism of gene extinction in the majority of cells of numerous living organisms, from plants to insects and up to mammals (Sharp, 2001). RNA interference prevents the production of a functional protein from a gene by leading to the destruction of the intermediate mRNA (Bass, 2000; Sharp, 2001). The siRNAs can be used in naked form or incorporated into a vector. Preferably, an interfering RNA that blocks GM3 synthase transcription can have the sequence GGGUUAUUCUGAACAUGUUtt (SEQ ID No: 5).

Aptamers can also be used to inhibit GM3 synthase transcription. Aptamers are oligonucleotide sequences that have the capacity to recognise virtually any class of target molecules with a high affinity and specificity. Such ligands can be isolated from a random sequence library by a screening method called SELEX (Systematic Evolution of Ligands by EXponential enrichment), as described in Tuerk and Gold (1990). The random sequence library can be obtained by DNA synthesis by means of combinatorial chemistry. In such a library, each member is a linear oligomer (optionally chemically modified) corresponding to a unique sequence. The possible modifications, applications and advantages of this class of molecules have been reviewed by Jayasena (1999).

In another embodiment, the method or use according to the invention involves using an inhibitor of the activity of the GM3 synthase protein. Inhibitors of GM3 synthase activity can easily be identified by screening methods, including cellular or biochemical tests in vitro, as described in the present patent application. An inhibitor can be of a peptide nature, a peptidomimetic or a non-peptide mimic (Rubin-Carrez, 2000), such as a small organic molecule capable of interfering with the enzymatic activity of GM3 synthase, for example by blocking or reducing the transfer of a sialic acid group from a donor to a sialic acid acceptor, and/or by blocking or reducing GM3 synthesis.

The GM3 synthase inhibitor can also be an antibody, particularly an inhibitor directed against the human GM3 synthase shown in the sequence SEQ ID No: 2 or the murine GM3 synthase shown in the sequence SEQ ID No: 4. Said antibodies can be polyclonal or monoclonal antibodies or fragments thereof, or chimeric antibodies, especially those which are humanised or immunoconjugated.

Polyclonal antibodies can be obtained by the customary procedures from the serum of an animal immunised against a protein. For example, the antigen used can be an appropriate peptide complex such as a complex of GM3 synthase coupled via a reactive residue to a protein (such as keyhole limpet haemocyanin, KLH) or another peptide. Rabbits are immunised with the equivalent of 1 mg of the peptide antigen according to the procedure described by Benoit et al. (1982). At four-week intervals the animals are treated with 200 µg injections of antigen and bled 10 to 14 days later. After the third injection, the antiserum is examined in order to determine its capacity to bind to the iodine-radiolabelled peptide antigen, prepared by the chloramine-T method, and is then purified by chromatography on a carboxymethyl cellulose (CMC) ion exchange column. The antibody molecules are then collected from the mammals and isolated to the desired concentration by the methods well known to those skilled in the art, for example by using DEAE Sephadex to obtain the IgG fraction. To increase the specificity of the polyclonal serum, the antibodies can be purified by immunoaffinity chromatography using immunising polypeptides in the solid phase. The antibody is brought into contact witch the immunising polypeptide in the solid phase for a sufficient time to cause the polypeptide to undergo an immune reaction with the antibody molecule in order to form an immunological complex in the solid phase.

Monoclonal antibodies can be obtained by the conventional lymphocyte fusion and hybridoma culture method described by Köhler and Milstein (1975). Other methods of preparing monoclonal antibodies are also known (Harlow et al., 1988). Monoclonal antibodies can be prepared by immunising a mammal (for example a mouse, rat or rabbit, or even a human, etc.) and using the lymphocyte fusion technique to produce hybridomas (Köhler and Milstein, 1975). There are alternatives to this customary technique. It is possible, for example, to produce monoclonal antibodies by expressing a nucleic acid cloned from a hybridoma. Antibodies can also be produced by the phage display technique by introducing antibody cDNAs into vectors, the latter typically being filamentous phages with V gene libraries on the surface of the phage (e.g. fUSE5 for E. coli, Scott and Smith, 1990). Protocols for constructing these antibody libraries are described in Marks et al. (1991).

The antibodies or antibody fragments of the invention can be e.g. chimeric antibodies, humanised antibodies or Fab and F(ab')₂ fragments. They can also take the form of immunoconjugates or labelled antibodies.

Aptamers constitute a class of molecules that represent an alternative to antibodies in terms of molecular recognition.

The inhibitors of the expression or activity of GM3 synthase can be formulated with one or more pharmaceutically acceptable excipients. As described earlier, these inhibitors can be a chemically synthesised compound, an antisense or interfering RNA or an anti-GM3 synthase antibody.

"Excipient" or "pharmaceutically acceptable vehicle" is understood as meaning any solvent, dispersion medium, absorption retarder, etc. that does not produce a secondary reaction, for example an allergic reaction, in humans or animals.

The dosage naturally depends on the active substance in question, the mode of administration, the therapeutic indication and the age and state of the patient. The dose of protein or antibody is preferably 0.1 to 250 mg/kg per day and particularly preferably 1 to 100 mg/kg per day. When the pharmaceutical compositions comprise nucleic acids, the doses of nucleic acid (sequence or vector) to be administered are also adapted in particular according to the mode of administration, the targeted pathological condition and the duration of the treatment. In general, if recombinant viruses are used, these are formulated and administered as doses of about 10⁴ to 10¹⁴ pfu/ml and preferably of 10⁶ to 10¹⁰ pfu/ml. The term "pfu" (plaque forming unit) corresponds to the infectivity of a viral solution and can be determined by infecting an appropriate cell culture and measuring the number of plaques of infected cells, generally after 48 hours. The techniques for determining the pfu titre of a viral solution are amply described in the literature.

If parenteral administration is envisaged, more particularly by injection, the compositions of the invention comprising the active principle(s) take the form of injectable solutions and suspensions packaged in ampoules or bottles for slow perfusion. Injection can be effected especially by the subcutaneous, intramuscular or intravenous route.

In the case of oral administration, the compositions of the invention take the form of gelatine capsules, effervescent tablets, coated or uncoated tablets, sachets, dragees, ampoules or solutions to be taken orally, microgranules or prolonged release forms.

The forms for parenteral administration are obtained in conventional manner by mixing the active principle(s) with buffers, stabilisers, preservatives, solubilisers, isotonic agents and suspending agents. Using the known techniques, these mixtures are subsequently sterilised and then packaged in the form of intravenous injections.

The buffers used by those skilled in the art may be those based on organic phosphate salts.

Examples of suspending agents include methyl cellulose, hydroxyethyl cellulose, hydroxypropyl cellulose, acacia and sodium carboxymethyl cellulose.

Moreover, stabilisers useful according to the invention are sodium sulfite and sodium metabisulfite, while preservatives which may be mentioned are sodium p-hydroxybenzoate, sorbic acid, cresol and chlorocresol. To prepare an oral solution or suspension, the active principles are dissolved or suspended in an appropriate vehicle with a dispersant, a humectant, a suspending agent (e.g. polyvinylpyrrolidone), a preservative (such as methylparaben or propylparaben), a taste corrector or a colourant.

To prepare microcapsules, the active principles are combined with appropriate diluents, appropriate stabilisers, agents promoting the prolonged release of the active substances, or any other type of additive to form a central core, which is then coated with an appropriate polymer (e.g. a water-soluble resin or a water-insoluble resin). The techniques known to those skilled in the art will be used for this purpose.

The resulting microcapsules are then optionally formulated into appropriate dosage units.

Administration by the ocular route can also be envisaged.

In that case the pharmaceutical composition of the invention takes the form of an ophthalmic composition for local administration to the eye, for example an eye lotion or ophthalmic cream.

The inhibitors can also be formulated as liposomes. Liposomes are formed from phospholipids, which are dispersed in an aqueous medium and spontaneously form multilamellar concentric bilayer vesicles. These vesicles generally have a diameter of 25 nm to 4 µm and can be sonicated, resulting in the formation of smaller unilamellar vesicles, with a diameter of 200 to 500 A, containing an aqueous solution in their core.

Liposomes can be particularly advantageous for the administration of an active principle to a precise cellular or tissular target. This can be done by chemically coupling the lipids to targeting molecules, such as targeting peptides (for example hormones), or antibodies.

### Screening method

The invention further relates to an *in vitro* method of screening or identifying compounds useful in the treatment and/or prevention of microvascular complications of diabetes, wherein the capacity of at least one test compound to inhibit GM3 synthase activity is evaluated, a decrease in the level of activity of this enzyme being indicative of a compound useful in the treatment and/or prevention of microvascular complications of diabetes, and wherein the microvascular complication of diabetes is diabetic nephropathy.

The test compound can be of any type. It can be a natural or synthetic compound or a mixture of such compounds. It can also be a structurally defined substance or a substance of unknown structure, for example a biological extract.

The level of GM3 synthase activity in the presence of the test compound can be compared with the control level of activity in the absence of the test compound.

In a first embodiment, the screening method comprises steps that consist in bringing at least one test compound into contact with a cell that expresses a GM3 synthase, and determining the capacity of said compound to inhibit, i.e. block or reduce, the intracellular synthesis of ganglioside GM3. A decrease in the level of synthesis of ganglioside GM3 in the cell, compared with a cell not exposed to the test compound, is indicative of a compound useful in the treatment and/or prevention of microvascular complications of diabetes, and wherein the microvascular complication of diabetes is diabetic nephropathy.

The cell can be a cell that expresses GM3 synthase endogenously, for example a retinal pericyte or a renal mesangial cell. The cell can also be a cell transfected in a transitory or stable manner to express a GM3 synthase, with the aid of vectors for expressing the product of the GM3 synthase gene. These cells can be obtained by introducing, into prokaryotic or eukaryotic host cells, a nucleotide sequence inserted in a vector containing a sequence coding for GM3 synthase, and then culturing said cells under conditions that allow the replication and/or expression of the transfected nucleotide sequence.

The DNA vector, for example a plasmid vector, containing a sequence coding for a GM3 synthase can be introduced into a host cell by any technique known to those skilled in the art. In particular, it is possible to introduce the DNA vector in a naked form, i.e. without the aid of any kind of vehicle or system which would facilitate transfection of the vector into the cells (EP 465 529). Other available techniques are those of microinjection, electroporation, calcium phosphate precipitation or formulation with the aid of nanocapsules or liposomes. Biodegradable polyalkyl cyanoacrylate nanoparticles are particularly advantageous. In the case of liposomes, the use of cationic lipids favours encapsulation of the nucleic acids, which are negatively charged, and facilitates fusion with the negatively charged cell membranes.

Alternatively, the vector can be in the form of a recombinant virus comprising, inserted in its genome, a nucleic acid sequence coding for a GM3 synthase. The viral vector can preferably be selected from an adenovirus, a retrovirus, particularly a lentivirus, an adeno-associated virus (AAV), a herpes virus, a cytomegalovirus (CMV), a vaccine virus, etc.

The implementation of these recombinant expression techniques is well known to those skilled in the art.

Examples of host cells include especially mammalian cells such as CHO, COS-7, 293 and MDCK cells, insect cells such as SF9 cells, bacteria such as E. coli, and yeast strains.

The level of expression can be evaluated by determining the level of transcription of the gene or the level of translation of the protein encoded by this GM3 synthase gene, either directly or via e.g. a reporter gene.

The most common tests for following the transcription (i.e. determining the level of transcription) of the target gene (in this case the GM3 synthase gene) or the reporter gene are based on the northern blotting technique. The tests for following the translation (i.e. determining the level of translation) of the GM3 synthase protein or the reporter protein can be based especially on immunoassay techniques or can use fluorimetric, luminescent or other techniques for detecting reporter proteins (green fluorescent protein, GFP; luciferase; chloramphenicol acetyltransferase, CAT; etc.).

Immunoassay techniques can be carried out according to various formats well known to those skilled in the art, for example by ELISA, radioimmunoassay, in situ immunoassays, western blotting, immunofluorescence, etc. The anti-GM3 synthase protein antibodies useful for detecting the GM3 synthase protein can be produced as described below.

In another embodiment, the screening method comprises steps that consist in bringing at least one test compound into contact with a natural, mutated or recombinant GM3 synthase or a GM3 synthase of biological origin, and determining the capacity of said compound to inhibit, i.e. block or reduce, the transfer of a sialic acid residue from a sialic acid donor to a 3-hydroxyl group of a galactose residue of a sialic acid acceptor. A decrease in the level of sialic acid transfer activity in the presence of said compound, compared with the level of sialic acid transfer in the absence of said compound, is indicative of a compound that inhibits GM3 synthase and is useful in the treatment and/or prevention of microvascular complications of diabetes, and wherein the microvascular complication of diabetes is diabetic nephropathy.

The level of GM3 synthase activity is advantageously evaluated by bringing a GM3 synthase into contact with a sialic acid donor and a sialic acid acceptor under appropriate conditions that allow the transfer of sialic acid from donor to acceptor. In general, "appropriate conditions" denote a reaction medium in which the catalytic reaction can occur. The medium can include e.g. buffers, oxidising and/or reducing agents and cofactors. The pH, temperature and ionic concentration of the medium are generally adjusted. Preferably, the GM3 synthase activity is evaluated in a medium buffered to a pH of between 6 and 7 and preferably of between 6.5 and 6.7, at a temperature of between 35 and 39°C and preferably of 37°C. The reaction is advantageously performed in a medium containing 10 mM Mn²⁺, for example 10 mM MnCl₂. GM3 synthase activity tests have been described especially in international patent application WO 97/47749, US patent 6555371 or the article by Wakarchuk et al. (1996).

Preferably, the GM3 synthase activity is evaluated by quantifying the transfer of sialic acid from a sialic acid donor, such as CMP-N-acetylneuraminate, to the 3-hydroxyl group of a galactose residue of lactosylceramide (LacCer) to form ganglioside GM3. A decrease in the disappearance of CMP-N-acetylneuraminate and/or lactosylceramide and/or in the formation of GM3 is indicative of a compound that inhibits GM3 synthase. A method according to the invention therefore comprises determining the level of sialic acid transfer from CMP-N-acetylneuraminate to lactosylceramide in the presence or absence of the test compound.

Advantageously, the sialic acid donor and/or acceptor are labelled in a detectable manner. The labelling can be effected by any appropriate technique well known to those skilled in the art. This can be e.g. radioactive, enzymatic, luminescent or fluorescent labelling or a combination of these techniques.

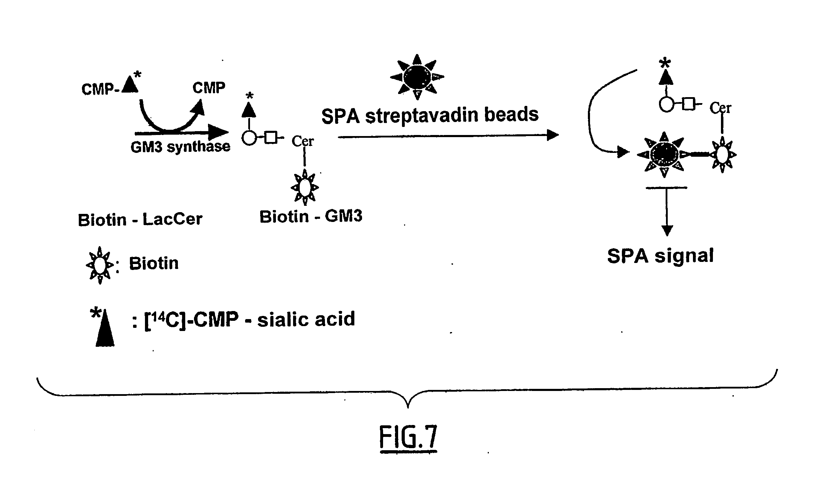

A preferred screening test according to the invention is described in Figure 7. In this test, GM3 synthase is brought into contact with [¹⁴C]-CMP-sialic acid and with lactosylceramide coupled to biotin. SPA^{®} technology (Amersham Biosciences) is based on the emission of β particles by the disintegration of certain radioactive elements. If the radioactive molecule is sufficiently close to an SPA scintillation bead, the radioactive disintegration stimulates the group of scintillants in the bead, producing a luminescent emission. The signal can be detected by a scintillation counter and/or a CCD imager. On the other hand, in the case of a free radioactive molecule in a solution containing SPA beads, i.e. a radioactive molecule that does not interact with SPA beads, the β emission associated with the disintegration of the radioactive molecule does not have sufficient energy to reach an SPA bead and no light emission is generated. In the context of the present test, measurement of the luminescent signal therefore reflects the amount of GM3 since, in the reaction medium, only this compound is associated with SPA beads via the biotin/streptavidin complex and carries a radioactive sialic acid group.

The Examples and Figures which follow illustrate the invention without implying a limitation.

### FIGURES

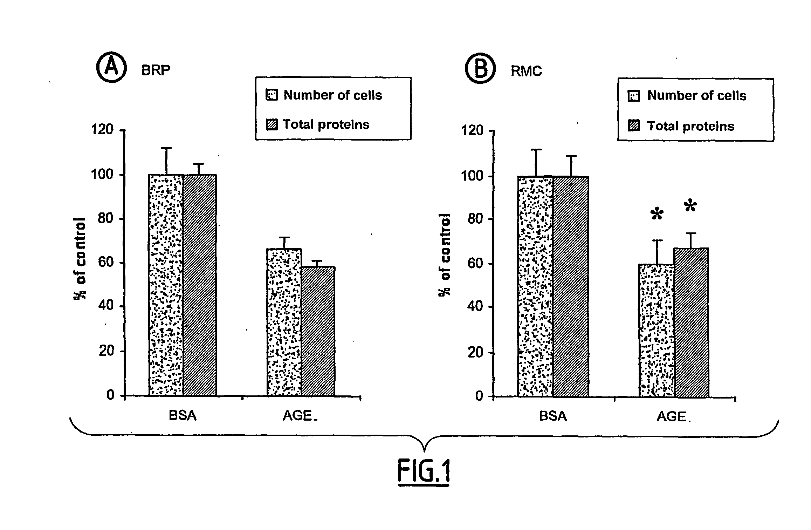

Figure 1 shows the inhibition of the proliferation of pericytes (BRP) and renal mesangial cells (RMC) by AGE. The cells were exposed to 3 µM BSA or AGE for 4 days (RMC) or 7 days (BRP). The cells were then trypsinised and counted using a haemocytometer, and the total amount of proteins was determined. The results are shown as a percentage of the BSA control and represent the mean ± SEM of 6 (BRP) or 9 (RMC) independent experiments, each performed in duplicate. *p < 0.05 against the BSA control.
Figure 2 shows the ganglioside biosynthesis route modified on the basis of the article by van Echten et al. (1993). Only series a and b gangliosides are detected in the BRP and RMC (surrounded gangliosides).

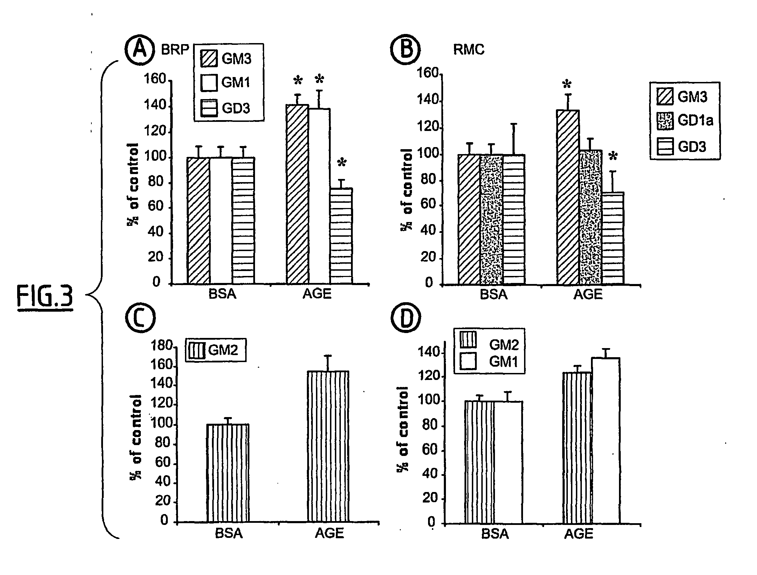

Figure 3 illustrates the modulation of the ganglioside profile in pericytes and mesangial cells. Pericytes (A) or mesangial cells (B) were exposed to 3 µM BSA or AGE for 4 or 7 days, respectively, and then harvested. The gangliosides were extracted, purified, analysed by HPTLC and developed by staining with resorcinol, as described in the section "Materials and methods". The results are expressed as a percentage of the BSA control and represent the mean ± SEM of 6 (BRP) or 9 (RMC) independent experiments, each performed in duplicate. *p < 0.05 against BSA control. In (C) and (D) the gangliosides were metabolically labelled with 1 µCi/ml of [¹⁴C]-galactose, extracted, separated by HPTLC and analysed by autoradiography. The results are expressed as a percentage of the BSA control and represent the mean ± SEM of three independent experiments.

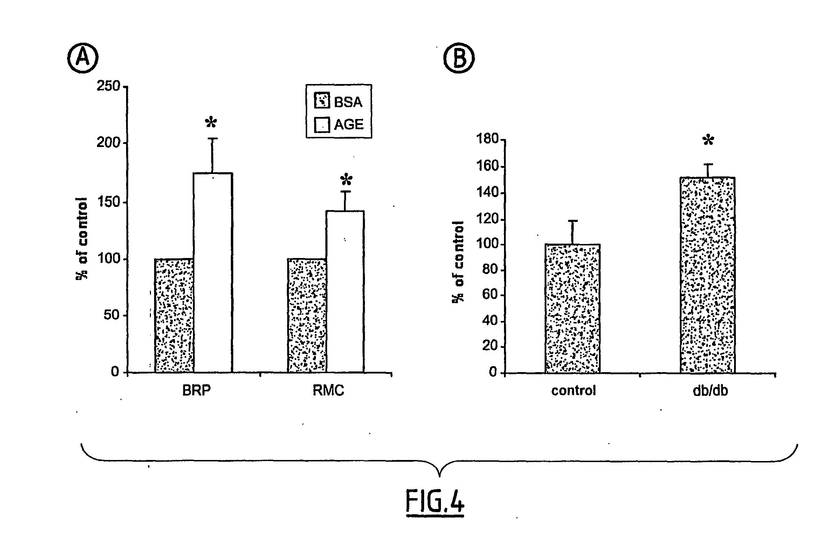

Figure 4 shows the increase in GM3 synthase activity in isolated glomeruli and cells caused by AGE. (A) The cells were treated with 3 µM BSA or AGE for 4 days (RMC) or 7 days (BRP). The GM3 synthase activity was measured on the cell homogenates as described in the section "Materials and methods". The control activity was 2.7 and 5.1 pmol/h/mg of proteins in the BRP and RMC, respectively. The results are expressed as a percentage of the BSA control and represent the mean ± SEM of 4 or 5 independent experiments. (B) The GM3 synthase activity was measured on the highly purified homogenates of glomeruli of control mice (db/m) and db/db mice. The control activity was 4.7 pmol/h/mg of proteins. The results are expressed as a percentage of the control and represent the mean ± SEM of 4-5 animals. *p < 0.05 against BSA control or control mice.

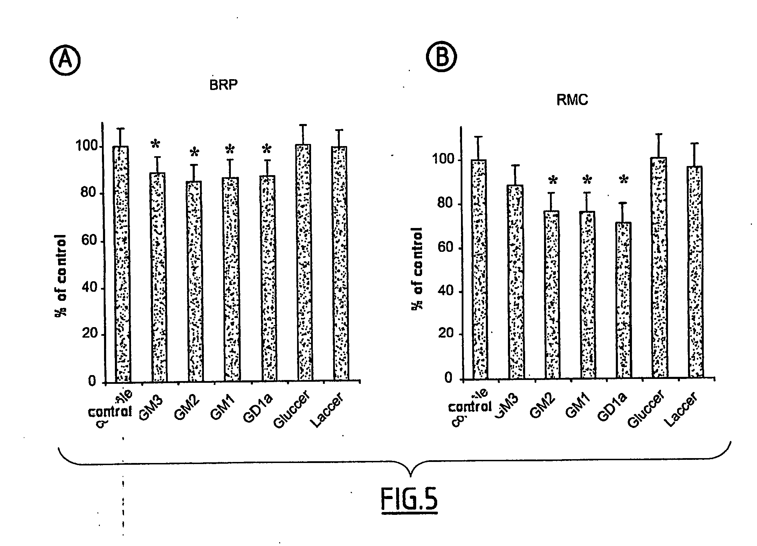

Figure 5 illustrates the inhibition of the cell proliferation caused by exogenous series a gangliosides. Pericytes (A) or mesangial cells (B) were treated with ganglioside-BSA complexes for 4 or 7 days, respectively. At the end of the treatment, the total proteins were measured. The results are expressed as a percentage of the BSA control and represent the mean ± SEM of 5-6 independent experiments, each performed in triplicate. *p < 0.05 against BSA control.

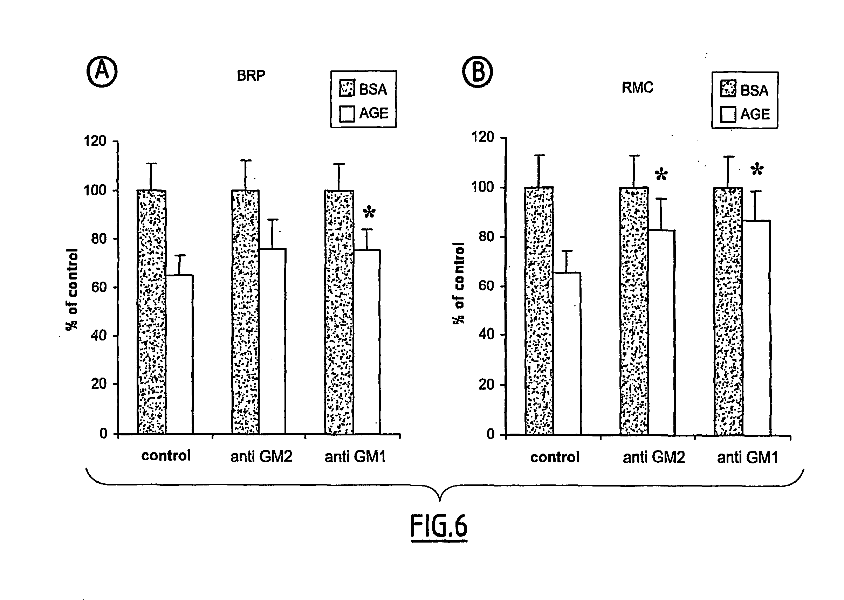

Figure 6 shows that anti-series a ganglioside GM2 and GM1 antibodies protect against the effects of AGE. Pericytes (A) or mesangial cells (B) were treated with 3 µM BSA or AGE in the presence or absence of 5 µg per well of anti-GM2 or anti-GM1 polyclonal antibodies. At the end of the treatment, the cells were washed and lysed and the total amount of proteins was measured. The results are expressed as a percentage of the BSA control and represent the mean ± SEM of 5-6 independent experiments, each performed in triplicate. *p < 0.05 against the cells treated with AGE.
Figure 7 illustrates the detection of GM3 synthase activity by a test which combines detection of the reaction product, ganglioside GM3, by scintigraphy and luminescence. GM3 synthase catalyses the transfer of labelled sialic acid from [¹⁴C]-CMP-sialic acid to lactosylceramide coupled to biotin (Biotin-LacCer). SPA (Scintillation Proximity Assay, Amersham Biosciences) beads coupled to streptavidin are then brought into contact with the resulting ganglioside GM3 coupled to biotin and labelled with ¹⁴C. The SPA signal resulting from the interaction between the GM3 radioactivity and the SPA beads is then measured.

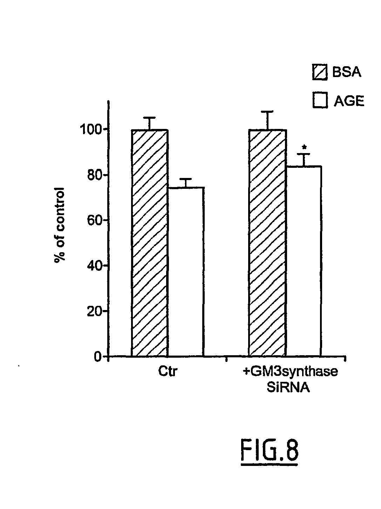

Figure 8 shows that transfection with GM3 synthase siRNA protects RMC. 24 hours after the transfection of RMC with 400 nM GM3 synthase siRNA, the cells were treated with 3 µM BSA control or AGE. At the end of the treatment, the total proteins were measured. The results are expressed as a percentage of the BSA control and represent the mean ± SEM of 6 independent experiments. *p < 0.05 against the cells treated with AGE.

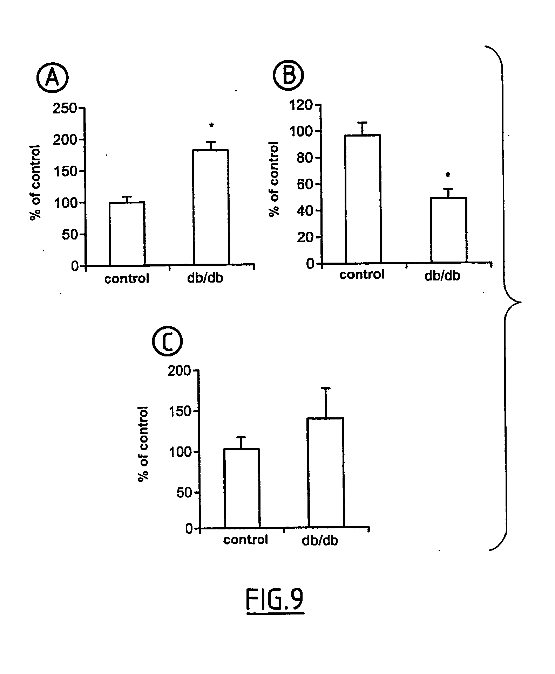

Figure 9 illustrates that GM3 and GD3 synthase activities and GM3 levels are modulated in the diabetic mouse renal cortex. The GM3 synthase activity (A) and GD3 synthase activity (B) were measured on homogenates of the renal cortex of control mice (db/m) and diabetic mice (db/db). The GM3 levels (C) in control mice (db/m) and diabetic mice (db/db) are shown. In the control mice, the GM3 levels were 66 ± 9 ng/ml of proteins. The results are expressed as a percentage of the BSA control and represent the mean ± SEM of 4-6 animals. *p < 0.05 against the cells treated with AGE.

### EXAMPLES

### Example 1 - Materials and methods

### Cell isolation and culture

Bovine retinal pericytes (BRP) were isolated from bovine retinal microvessels as described previously (Lecomte et al., 1996). Briefly, the retinas were obtained by dissection under sterile conditions from enucleated bovine eyes obtained from a local abattoir. After the contaminating pigmented epithelial cells of the retina had been removed, the retinas (2 per culture dish) were sliced into small pieces and homogenised in a Dounce homogeniser in a Hanks balanced saline solution (Ca²⁺/Mg²⁺-free oxygenated HBSS supplemented with Hepes 10 mM, pH 7.4, antibiotics 1%, bovine serum albumin (BSA, Sigma, Saint-Quentin Fallavier, France) 0.5%). The homogenates were centrifuged at 1000 g for 5 minutes at 4°C and the residues were resuspended in an enzymatic solution containing collagenase/ dispase (Roche Diagnostics, Mannheim, Germany) (1 mg/ml in a Ca²⁺/Mg²⁺-free oxygenated HBSS, Hepes 10 mM, pH 7.4, antibiotics 1%, DNase 20 U/ml and Nα-tosyllysine chloromethyl ketone (TLCK) 150 ng/ml, Sigma). After digestion (20 minutes at 37°C), the microvessel fragments were placed on a 40 µm nylon filter and deposited in 6 cm dishes covered with fibronectin. The primary cultures were cultivated in a DMEM (Dulbecco modified Eagle's medium) supplemented with 10% of foetal bovine serum (Gibco, Invitrogen Corporation, N.Y., United States), 1% of glutamine and 1% of penicillin/streptomycin (Sigma), and the medium was replaced every two days. After the adhesion period, an excrescence of pericytes from the microvessels occurred after 48 hours and the cells reached confluence in about 10 days. The BRP cultures were 100% pure, as characterised by their polygonal irregular morphology with pseudopodia and their growth into non-apposed cells, and as evaluated by positive labelling for both α₁-actin and a specific glycolipid antigen (3G5 antibody) and by negative labelling for the von Willebrand factor expressed in the endothelial cells (Lecomte et al., 1996). The cells were passaged with trypsin-EDTA (Sigma) (1:3) and culture was continued with the same medium up to the second passage, during which the BRP were treated.

Rat renal mesangial cells (RMC) were obtained from purified glomeruli of young male Wistar rats (Charles River, l'Arbresle, France). Briefly, cortex fragments were isolated from rat kidneys freshly removed under sterile conditions. Small pieces were mechanically forced through a 230 µm mesh with HBSS buffer. The glomeruli, passed through this mesh, were then forced through a 73.7 µm mesh. Finally, they were placed on a 70 µm mesh and placed in 6 cm dishes covered with fibronectin (4 dishes for 2 renal glomeruli) in DMEM supplemented with 20% of foetal bovine serum, 1% of glutamine and 1% of penicillin/streptomycin. After the attachment period, the excrescence of RMC from glomeruli occurred after three weeks and the cells were then cultivated to the fifth passage in order to remove the residual epithelial and endothelial cells. The RMC are characterised by morphological criteria (star shape, wick shape when they are confluent) and by positive labelling with vimentin, smooth muscle α-actin and Thy-1 antigen. The cells were then cultivated in DMEM supplemented with 15% of foetal calf serum, 1% of glutamine and 1% of penicillin/streptomycin. They were used between the fifth and fifteenth passages.

### Isolation of mouse glomeruli

Highly purified glomeruli were obtained from diabetic (db/db) or control (db/m) eleven-week-old mice (Charles River) by the magnetic bead perfusion technique, as described by Takemoto et al. (Takemoto et al., 2002). Briefly, the mice were perfused through the heart with a Dynabeads solution (Dynal, Compiègne, France) and the kidneys were then removed, finely sliced and digested with collagenase (Roche Diagnostics). After filtration, the glomeruli which had accumulated beads in their capillaries were retained by magnetism and then washed twice before homogenisation. This technique provides pure preparations of glomeruli with a low degree of tissue contamination.

### Isolation of mouse renal cortices

Renal cortex fragments were isolated from the kidneys of diabetic (db/db) or control (db/m) eleven-week-old mice (Charles River). Briefly, the animals were anaesthetised and sacrificed and the kidneys were removed. The renal cortex fragments were then obtained by dissection and mechanically homogenised with a Dounce homogeniser in a 25 mM Hepes buffer containing 1 mM EDTA and 10 µl/ml of protease inhibitors.

### Preparation of AGE

AGE were prepared by incubating bovine serum albumin (final concentration of 7.2 mg/ml) (Sigma) with 100 mM methylglyoxal (Sigma) at 37°C for 50 hours. Bovine serum albumin (BSA) was incubated under the same conditions in the absence of methylglyoxal and used as a control preparation (BSA control). The AGE and the BSA control were purified on PD10 Sephadex G25 columns (Amersham Biosciences, Uppsala, Sweden) to remove the salts and unreacted carbonyls, and were then sterilised by filtration and kept at -20°C until used.

### Treatment with AGE

The AGE and the BSA control (final concentration of 3 µM) were added to the culture medium 24 hours after inoculation. Each cell type was treated for one passage (about 7 days for the BRP and 4 days for the RMC). The culture medium was replaced with a fresh medium every two days.

### Measurement of cell growth

At the end of the treatment, the cells were harvested with trypsin and the cell residues were washed twice with a phosphate buffered saline solution (iced PBS) (Sigma). For each sample, one aliquot of cells was counted using a haemocytometer in order to determine the number of cells. Another aliquot was used to measure the proteins by the Bradford technique.

### Analysis of gangliosides

For metabolic labelling of the gangliosides, 0.2 µCi/ml or 1 µCi/ml of [¹⁴C(U)]-D-galactose (329.5 mCi/mmol) (PerkinElmer Life Sciences, Boston, MA) was added to the culture medium overnight for the labelling experiments with a high specific activity (GM2 and GM1 measuring experiment). The cells (5-8 x 10⁵ pericytes or 12-20 x 10⁵ mesangial cells) were then harvested by trypsinisation and washed three times in PBS. The gangliosides were extracted from the cell residues by the method described by Bouchon et al. (Bouchon et al., 1990), modified by Natalizio et al. (Natalizio et al., 2002). Briefly, the cell residues were dispersed in 2 ml of chloroform (C)/methanol (M) (1:1, v/v), mixed vigorously and extracted overnight at 4°C. After centrifugation, the residues were extracted twice with 2 ml of the same solvent. The pooled extracts of total lipids were dried by evaporation and separated with the aid of a 1 mM C/M/PBS solution (10:10:7, v/v/v). The upper phases, containing the gangliosides, were then desalted on a C18 silica gel column (Waters Corporation, Milford, MA) and analysed by HPTLC (Merck, Darmstadt, Germany). The plates were developed in 0.2% C/M/CaCl₂ (55:45:10, v/v/v). The gangliosides were visualised by autoradiography using a phosphorus screen and a Storm 820 (Molecular Dynamics, Amersham Pharmacia Biotech, Piscataway, United States) and by labelling with resorcinol (specific stain for gangliosides: resorcinol 0.3% (Sigma), CuSO₄ 0.03%, HCl 30%) using an Image Master VDS-CL (Amersham Pharmacia Biotech). Quantification was performed using an Image Quant (Molecular Dynamics). As GT1 b was absent from the ganglioside profiles of both the BRP and the RMC, it was added to the samples as an internal standard prior to lipid extraction.

### Measurement of GM3 synthase activity

At the end of the treatment, the cells were washed with PBS, incubated for 20 minutes at 4°C in 50 µl of lysis buffer (20 mM sodium cacodylate, pH 6.6 (Sigma), 0.2% of Triton X-100, 1 mM EDTA, 10 µl/ml of protease inhibitors, Calbiochem, La Jolla, California, United States) and then collected by scraping. Four dishes of BRP (about 2-3 x 10⁶ cells) and three dishes of RMC (about 3-6 x 10⁶ cells) were pooled. The cell lysates were centrifuged at 10,000 g for 5 minutes and the proteins in the supernatants were used to measure the GM3 synthase activity. The glomerular residues were mechanically homogenised with a syringe in 25 mM Hepes containing 1 mM EDTA and 10 µl/ml of protease inhibitors. The homogenates were then centrifuged at 1000 g for 2 minutes and the postnuclear supernatant was used to measure the GM3 synthase activity. Equivalent amounts of proteins for each sample (about 500 µg for the cells and 100 µg for the glomeruli) were used to perform the test. The samples were mixed with an equal volume of reaction buffer containing final concentrations of 0.1 mM lactosylceramide (Matreya, Biovalley, Marne la Vallée, France), 4 µCi/ml of [sialic-4,5,6,7,8,9-¹⁴C]-CMP-sialic acid (325.2 mCi/mmol) (PerkinElmer Life Sciences), 100 µM CMP-sialic acid (Sigma), 10 mM MgCl₂, 0.2% of Triton X-100 and 100 mM sodium cacodylate, pH 6.6. After agitation, the reaction mixtures were incubated at 37°C for 50 minutes. The reactions were stopped by loading the samples onto silica gel 60 columns (Merck) to separate the excess substrates from the products. After the columns had been washed with water, the gangliosides were eluted with C/M (1:1, v/v) and the solvent was dried under nitrogen. Finally, the gangliosides were separated by thin layer chromatography (Merck) and the reaction products were developed by autoradiography. The GM3 synthase activity was expressed in pmol of GM3 produced/h/mg of proteins.

### Treatment with exogenous gangliosides

To evaluate the effect of exogenous gangliosides on the proliferation of BRP and RMC, the cells were cultivated in 96-well plates. Exogenous glycolipids GM3, GM2, GM1 and GD1a, glucosylceramide and lactosylceramide (Matreya) were added to the complete culture medium in a final concentration of 50 µM in the form of complexes with BSA in a 1:1 ratio in DMEM/10 mM Hepes, pH 7.4. At the end of the treatment, the cells were washed twice with PBS and lysed for 30 minutes at 37°C in 50 µl of a Ripa lysis buffer (PBS 10 mM, NP40 1% (Pierce, Perbio Science, Brebières, France), sodium deoxycholate 0.5%, SDS 0.1%, protease inhibitors 10 µl/ml). As the number of cells in our experiments was correlated with the total protein concentrations (cf. Fig. 1), the total proteins were measure using the BCA protein test (Pierce) in order to evaluate the cell proliferation.

### Treatment with anti-series a ganglioside antibodies

To block the potential effects of series a gangliosides, the cells were cultivated in 96-well plates and treated with 3 µM AGE or control BSA in the presence or absence of 50 µlg/ml of anti-GM2 polyclonal antibody (Calbiochem) or anti-GM1 polyclonal antibody (Matreya). At the end of the treatment, the cells were washed twice with PBS and lysed in 50 µl of Ripa lysis buffer and the proteins were measured in order to quantify the cell proliferation.

### Transfection of RMC with a GM3 synthase siRNA

To block the GM3 synthase activity, RMC were cultivated in 6-well culture plates up to 30% confluence and then transfected with an interfering RNA (siRNA) specific for GM3 synthase, which was designed against a rat cDNA sequence (Ambion, Huntingdon, UK) using an Oligofectamine reagent (Invitrogen Corporation). The GM3 synthase antisense sequence used was GGGUUAUUCUGAACAUGUUtt (SEQ ID No: 5). In preliminary experiments, a dose-response study was carried out by transfecting the cells with increasing concentrations of siRNA (0-800 nM). After 72 h of transfection, the GM3 synthase activity was measured on homogenates of transfected cells. The proliferation was then evaluated on the cells transfected with the siRNA. For this purpose, 24 hours after transfection with 400 nM siRNA, the cells were treated with 3 µM BSA control or AGE (3 days). The cells were then washed twice with PBS and lysed in a Ripa lysis buffer and the proteins were measured in order to evaluate the cell proliferation.

### Statistical analysis

The data are expressed as means ± SEM and presented as a percentage of the controls. In the cell studies, the Wilcoxon rank test was used to evaluate the significance of the difference between the groups. The Student t-test was used for the GM3 synthase activity in the experiments on mice. p < 0.05 was considered to be statistically significant.

### Example 2 - Results

### 1 - AGE inhibit the proliferation of pericytes and mesangial cells

To compare the effect of AGE on the proliferation of pericytes and mesangial cells, the cells were treated with BSA or AGE at a concentration of 3 µM for 4 to 7 days. The cell counts showed that AGE reduced the number of pericytes and mesangial cells by 33 and 40%, respectively (Fig. 1). The total proteins were also measured and were found to have decreased in correlation with the number of cells. These results demonstrate that AGE have similar adverse effects on the proliferation of both pericytes and mesangial cells and enabled the inventors to elucidate the common mechanisms involved in the response to AGE in both these cell types.

### 2 - AGE increase series a gangliosides in pericytes and mesangial cells

The inventors' previous results suggested that AGE could modulate the ganglioside profile in retinal microvascular cells (Natalizio et al., 2001). The ganglioside profiles were analysed in BRP and RMC in response to the BSA control or the AGE. The ganglioside profile is specific for the cell type. Under control conditions, the main gangliosides in the pericytes were series a gangliosides GM3 (63% of the total gangliosides detected) and GM1 (9%) and series b ganglioside GD3 (28%). The profile in the mesangial cells differed by the very small amount of GD3 (5%) and, in the case of series a, by the presence of GD1 a (20%), GM3 still being the main ganglioside (75%).

An increase in series a gangliosides and a decrease in series b gangliosides were observed in both cell types treated with AGE (Fig. 3). In the pericytes, series a gangliosides GM3 and GM1 were increased by about 40%, whereas series b ganglioside GD3 was reduced by 24% (Fig. 3A). In the mesangial cells, GM3 was increased by 33%, whereas GD3 was reduced by 30%; the GD1a levels were not affected (Fig. 3B). Similar results were obtained by autoradiography after labelling with galactose. As series a gangliosides GM2 in the BRP and GM2 and GM1 in the RMC were difficult to detect by labelling with resorcinol, the cells were labelled with [¹⁴C]-D-galactose of high specific activity and the gangliosides were then analysed in the control cells and the cells treated with AGE. The results showed that GM2 was increased by 55% in the BRP (Fig. 3C) and that GM2 and GM1 were increased by 25 to 35% in the RMC (Fig. 3D). These results indicated that AGE induce similar modifications to the ganglioside profiles in both pericytes and mesangial cells. They also suggest that the increase in series a gangliosides may be a common mechanism underlying the decrease in cell proliferation.

### 3 -AGE increase GM3 synthase activity in pericytes and mesangial cells

In an attempt to explain the mechanism responsible for the observed increase in series a gangliosides, the activity of GM3 synthase, the limiting enzyme for the synthesis of series a gangliosides, was measured in the control and treated cells. The results presented in Figure 4 show that the treatment with AGE increased the GM3 synthase activity by a factor of about 1.8 in the pericytes and of about 1.5 in the mesangial cells, very probably by increasing the maximum rate of the enzymatic reaction. These results suggest that AGE exert common mechanisms in RMC and BRP by regulating the GM3 synthase.

### 4 - Exogenous series a gangliosides inhibit the proliferation of pericytes and mesangial cells

The inventors studied whether the exogenous addition of series a gangliosides would affect the proliferation of pericytes and mesangial cells. The cells were treated for one passage (7 days for the BRP and 4 days for the RMC) with 50 µM gangliosides and, as control, with the non-sialylated glucosylceramide and lactosylceramide precursors. Figure 5 shows that GM2, GM1 and GD1a inhibited the proliferation of the pericytes and mesangial cells most effectively (about 15 to 30%). GM3 weakly inhibited the proliferation of the pericytes, but had no significant effect on the mesangial cells. Glucosylceramide and lactosylceramide, used as control, had no effect. These results indicate that series a gangliosides inhibit the proliferation of pericytes and mesangial cells. In particular, GM2 and GM1 are increased in BRP and RMC in response to AGE and they degrade the proliferation of both these cell types; these series a gangliosides could therefore be the common mediators of the AGE effect.

### 5 - Anti-series a ganglioside antibodies protect pericytes and mesangial cells against inhibition of the proliferation caused by AGE

To study whether GM2 and GM1 mediate the effects of AGE, the cells were treated with AGE in the presence of anti-GM1 and anti-GM2 antibodies. In the control cells treated with BSA, the proliferation did not differ in the presence or absence of the anti-ganglioside antibodies. Treatment of the cells with AGE in the presence of anti-GM2 and anti-GM1 antibodies partially prevented the decrease in the proliferation of pericytes and mesangial cells (Fig. 6). In the light of the effects of exogenous gangliosides, these observations suggest that series a gangliosides, particularly GM1 and GM2, are the common mediators of the inhibition of the proliferation induced by AGE in pericytes and mesangial cells.

### 6 - GM3 synthase activity is increased in diabetic mouse glomeruli

To evaluate the effect of a diabetic environment on GM3 synthase activity *in vivo*, the enzymatic activity was measured in highly purified glomeruli of a db/db diabetic mouse model. The results presented in Figure 4B show that the GM3 synthase activity was increased by 50% in the glomeruli of diabetic cells, compared with the controls (db/m).

### 7 - A GM3 synthase siRNA partially protects from effects induced by AGE

To elucidate in greater detail the role of series a gangliosides in the mediation of AGE effects, RMC were transfected with a GM3 synthase siRNA. Preliminary experiments showed that the siRNA effectively inhibited GM3 synthase activity in the RMC. The proliferation of the transfected cells and treated cells was then measured. As shown in Figure 8, the effects of AGE on the proliferation of the RMC are partially inhibited in the cells transfected with the GM3 synthase siRNA. These results clearly demonstrate the involvement of gangliosides in the mediation of AGE effects. The partial nature of the effects can be explained by the fact that: (i) the AGE effects were less potent than in the previous experiments, given that the cells were treated to a more advanced level of confluence so as to increase the efficacy of transfection, and (ii) the GM3 synthase activity was inhibited by only 50%.

### 8 - GM3 and GD3 synthase activities and GM3 levels are modified in diabetic mouse renal cortex

To evaluate ex *vivo* the effect of a diabetic environment on the ganglioside biosynthesis pathway, the GM3 and GD3 synthase activities were measured on homogenates of renal cortex of the db/db diabetic mouse model. The results presented in Figure 9A-B show that the GM3 synthase activity was increased by 80%, whereas the GD3 synthase activity was reduced by 50% in the diabetic mouse renal cortices, compared with the controls (db/m). The gangliosides were also analysed and showed an increase, albeit not statistically significant, in the GM3 levels in the renal cortex of db/db diabetic mice (Figure 9C). Overall, these results therefore demonstrate the physiopathological sense of the results obtained in RMC and BRP treated with AGE.

### REFERENCES

Bass, Cell, 2000, 101, 235-238
Benoit et al. (1982) PNAS USA, 79, 917-921
Bouchon et al. (1990) Biochim. Biophys. Acta, 1051, 1-5
Cech T.R. (1989) RNA as an enzyme. Biochem. Int., 18, 7-14
Felgner P.L., Ringold G.M. (1989) Cationic liposome-mediated transfection. Nature, 337, 387-8
Fong et al. (2003) Diabetes Care, 26, 226-229
Forrester et al. (1997) Pathogenesis of diabetic retinopathy and cataract. In Textbook of diabetes. Pickup J., Williams G., Eds Oxford, 45.1-45.19
Fraley R., Subramani S., Berg P., Papahadjopoulos D. (1980) Introduction of liposome-encapsulated SV40 DNA into cells. J. Biol. Chem., 255, 10431-5
Harlow E. et al. (1988) Antibodies: A Laboratory Manual, Cold Spring Harbor Laboratory, New York
Hakomori et al. (1990) J. Biol. Chem., 265, 18713-18716
Hatanaka Y. et al. (1996) Synthesis and characterisation of a carbene-generating biotinylated lactosylceramide analog as a novel chromogenic photoprobe for GM3 synthase. Chem. Pharm. Bull. (Tokyo), 44(5), 1111-4
Hofmann et al. (1999) Cell, 97, 889-901
Inokuchi J. et al. (1998) A synthetic ceramide analog (L-PDMP) up-regulates neuronal function. Ann. NY Acad. Sci., 845, 219-24
Jayasena (1999) Aptamers: an emerging class of molecules that rival antibodies in diagnostics. Clin. Chem., 45(9), 1628-50
Kaneda Y., Iwai K., Uchida T. (1989) Increased expression of DNA co-introduced with nuclear protein in adult rat liver. Science, 243, 375-8
Krolewski et al. (1997) Clinical features and epidemiology of diabetic nephropathy. In Textbook of diabetes, Pickup J., Williams G., Eds Oxford, 53.1-53.13
Köhler and Milstein (1975) Nature, 256, 495-497
Lal et al. (2002) Kidney Int., 61, 2006-2014
Lecomte et al. (1996) J. Neurochem., 66, 2160-2167
Marks et al. (1991) J. Mol. Biol., 222, 581-597
Natalizio et al. (2001) Biochem. Biophys. Res. Commun., 281, 78-83
Pagano J.S., McCutchan J.H., Vaheri A. (1967) Factors influencing the enhancement of the infectivity of poliovirus ribonucleic acid by diethylaminoethyldextran. J. Virol., 1, 891-7
Rippin J.D., Barnett A.H., Bain S.C. (2004) Cost-effective strategies in the prevention of diabetic nephropathy. Pharmacoeconomics, 22(1), 9-28
Rubin-Carrez C. (2000). Les mimes peptidiques (Peptide mimics). Le technoscope, Biofutur, vol. 199
Sambrook, Fritsch & Maniatis, Molecular Cloning: A Laboratory Manual, Second Edition (1989) Cold Spring Harbor Laboratory Press, Cold Spring Harbor, New York
Schmidt et al. (1994) Arterioscler. Thromb., 14, 1521-1528
Schmidt et al. (1995) J. Clin. Invest., 96, 1395-1403
Sharp, Genes Dev., 2001, 15, 485, 49
Singh et al. (2001) Diabetologia, 44, 129-146
Scott J.K. and Smith G.P., Science, 1990, 249, 386-390
Stitt et al. (2003) Exp. Mol. Pathol., 75, 95-108
Takemoto et al. (2002) Am. J. Pathol., 161, 799-805
Tuerk C. and Gold L. (1990) Systematic evolution of ligands by exponential enrichment: RNA ligands to bacteriophage T4 DNA polymerase. Science, 3, 249, 505-10
Van Echten et al. (1993) J. Biol. Chem., 268, 5341-5344
Wakarchuk et al. (1996) Functional relationships of the genetic locus encoding the glycosyltransferase enzymes involved in expression of the lacto-N-neotetraose terminal lipopolysaccharide structure in Neisseria meningitidis. J. Biol. Chem., 271 (32), 19166-73
Wautier et al. (2001) Diabetes Metab., 27, 535-542
Wolf et al. (2000) Kidney Int. Suppl., 77, S59-S66

## Claims

1. Use of a GM3 synthase inhibitor for the manufacture of a drug intended for the treatment of a microvascular complication of diabetes, wherein the inhibitor is selected from the group consisting of antisense nucleic acids, ribozymes, interfering RNAs, aptamers, and a monoclonal or polyclonal antibody directed against human GM3 synthase and wherein the microvascular complication of diabetes is diabetic nephropathy.

2. Use according to Claim 1, wherein the inhibitor is an inhibitor of the expression of the GM3 synthase gene or protein.

3. Use according to any of claims 1 to 2, wherein the inhibitor is an antisense nucleic acid sequence that hybridises specifically with the sequence SEQ ID No: 1 under highly stringent conditions.

4. Use according to Claim 1, wherein the inhibitor is an inhibitor of GM3 synthase activity.

5. *In vitro* method of screening or identifying compounds useful in the treatment and/or prevention of microvascular complications of diabetes, wherein the capacity of at least one test compound to inhibit GM3 synthase activity is evaluated, a decrease in the level of GM3 synthase activity being indicative of a compound useful in the treatment and/or prevention of microvascular complications of diabetes and wherein the microvascular complication of diabetes is diabetic nephropathy.

6. Method according to Claim 5, said screening method comprising steps that consist in bringing at least one test compound into contact with a cell that expresses a GM3 synthase, and determining the capacity of said compound to inhibit the intracellular synthesis of ganglioside GM3, a decrease in the level of synthesis of ganglioside GM3 in the cell being indicative of a compound useful in the treatment and/or prevention of microvascular complications of diabetes, wherein the microvascular complication of diabetes is diabetic nephropathy.

7. Method according to any one of Claims 5 to 6, said screening method comprising steps that consist in bringing at least one test compound into contact with a GM3 synthase and determining the capacity of said compound to inhibit the transfer of a sialic acid residue from a sialic acid donor to a 3-hydroxyl group of a galactose residue of a sialic acid acceptor, a decrease in the level of sialic acid transfer activity being indicative of a compound that inhibits GM3 synthase and is useful in the treatment and/or prevention of microvascular complications of diabetes, wherein the microvascular complication of diabetes is diabetic nephropathy..

8. Method according to Claim 7, wherein the level of sialic acid transfer from CMP-N-acetylneuraminate to lactosylceramide is determined.

9. Method according to Claim 7 or 8, wherein the sialic acid donor and/or acceptor are labelled in a detectable manner.

## Patentansprüche

1. Verwendung eines GM3-Synthase-Inhibitors zur Herstellung eines Arzneimittels zur Behandlung einer mikrovaskulären Komplikation von Diabetes, wobei der Inhibitor aus der Gruppe ausgewählt wird, bestehend aus Antisense-Nukleinsäuren, Ribozymen, interferierenden RNA, Aptameren und einem monoklonalen oder polyklonalen Antikörper, der gegen humane GM3-Synthase gerichtet ist, und wobei die mikrovaskuläre Komplikation von Diabetes diabetische Nephropathie ist.

2. Verwendung nach Anspruch 1, wobei es sich bei dem Inhibitor um einen Inhibitor der Expression des GM3-Synthase-Gens oder -Proteins handelt.

3. Verwendung nach einem der Ansprüche 1 bis 2, wobei der Inhibitor eine Antisense-Nukleinsäuresequenz ist, die unter hochstringenten Bedingungen spezifisch mit der Sequenz SEQ ID NR: 1 hybridisiert.

4. Verwendung nach Anspruch 1, es sich bei dem Inhibitor um einen Inhibitor der GM3-Synthase-Aktivität handelt.

5. *In vitro*-Verfahren zum Screening oder zur Identifizierung von Verbindungen, die zur Behandlung und/oder Vorbeugung mikrovaskulärer Komplikationen von Diabetes geeignet sind, wobei die Fähigkeit mindestens einer Testverbindung, die GM3-Synthase-Aktivität zu hemmen, untersucht wird, wobei eine Abnahme im Spiegel der GM3-Synthase-Aktivität darauf hinweist, dass die Verbindung zur Behandlung und/oder Vorbeugung mikrovaskulärer Komplikationen von Diabetes geeignet ist, und wobei die mikrovaskuläre Komplikation von Diabetes diabetische Nephropathie ist.

6. Verfahren nach Anspruch 5, wobei das Screening-Verfahren Schritte umfasst, bei denen man mindestens eine Testverbindung mit einer Zelle in Kontakt bringt, die eine GM3-Synthase exprimiert, und die Fähigkeit der Verbindung bestimmt, die intrazelluläre Synthese von Gangliosid GM3 zu hemmen, wobei eine Abnahme im Spiegel der Synthese von Gangliosid GM3 in der Zelle darauf hinweist, dass die Verbindung zur Behandlung und/oder Vorbeugung mikrovaskulärer Komplikationen von Diabetes geeignet ist, wobei die mikrovaskuläre Komplikation von Diabetes diabetische Nephropathie ist.

7. Verfahren nach einem der Ansprüche 5 bis 6, wobei das Screening-Verfahren Schritte umfasst, bei denen man mindestens eine Testverbindung mit einer GM3-Synthase in Kontakt bringt und die Fähigkeit der Verbindung bestimmt, die Übertragung eines Sialinsäurerests von einem Sialinsäuredonor auf eine 3-Hydroxylgruppe eines Galactoserests eines Sialinsäureakzeptors zu hemmen, wobei eine Abnahme im Spiegel der Sialinsäureübertragungsaktivität darauf hinweist, dass die Verbindung GM3-Synthase hemmt und zur Behandlung und/oder Vorbeugung mikrovaskulärer Komplikationen von Diabetes geeignet ist, wobei die mikrovaskuläre Komplikation von Diabetes diabetische Nephropathie ist.

8. Verfahren nach Anspruch 7, wobei der Spiegel der Sialinsäureübertragung von CMP-N-Acetylneuraminat auf Lactosylceramid bestimmt wird.

9. Verfahren nach Anspruch 7 oder 8, wobei der Sialinsäuredonor und/oder -akzeptor auf nachweisbare Weise markiert werden.

## Revendications

1. Utilisation d'un inhibiteur de la GM3 synthase pour l'élaboration d'un médicament destiné au traitement d'une complication microvasculaire du diabète, dans laquelle l'inhibition est choisie dans le groupe constitué par les acides ncléiques antisens, les ribozymes, les ARN d'interférence, les aptamères, et un anticorps monoclonal ou polyclonal dirigé contre la GM3 synthase humaine et dans laquelle la complication microvasculaire du diabète est la néphropathie diabétique.

2. Utilisation selon la revendication 1, dans laquelle l'inhibiteur est un inhibiteur de l'expression du gène ou de la protéine de GM3 synthase.

3. Utilisation selon l'une quelconque des revendications 1 à 2, dans laquelle l'inhibiteur est une séquence d'acide nucléique antisens qui s'hybride spécifiquement avec la séquence SEQ ID No : 1 dans des conditions hautement stringentes.

4. Utilisation selon la revendication 1, dans laquelle l'inhibiteur est un inhibiteur de l'activité de la GM3 synthase.

5. Méthode *in vitro* de criblage ou d'identification de composés utiles dans le traitement et/ou la prévention de complications microvasculaires du diabète, dans laquelle on évalue l'aptitude d'au moins un composé à tester à inhiber l'activité de la GM3 synthase, une diminution du taux de l'activité de la GM3 synthase indiquant un composé utile dans le traitement et/ou la prévention de complications microvasculaires du diabète et dans laquelle la complication microvasculaire du diabète est la néphropathie diabétique.

6. Méthode selon la revendication 5, ladite méthode de criblage comprenant des étapes consistant à mettre en contact au moins un composé à tester avec une cellule qui exprime une GM3 synthase, et à déterminer l'aptitude dudit composé à inhiber la synthèse intracellulaire du ganglioside GM3, une diminution du taux de synthèse du ganglioside GM3 dans la cellule indiquant un composé utile dans le traitement et/ou la prévention de complications microvasculaires du diabète, dans laquelle la complication microvasculaire du diabète est la nphropathie diabétique.

7. Méthode selon l'une quelconque des revendications 5 à 6, ladite méthode de criblage comprenant des étapes consistant à mettre en contact au moins un composé à tester avec une GM3 synthase, et à déterminer l'aptitude dudit composé à inhiber le transfert d'un reste d'acide sialique à partir d'un donneur d'acide sialique vers un groupement 3-hydroxyle d'un reste galactose d'un accepteur d'acide sialique, une diminution du taux de l'activité de transfert d'acide sialique indiquant un composé inhibant la GM3 synthase et utile dans le traitement et/ou la prévention de complications microvasculaires du diabète, dans laquelle la complication microvasculaire du diabète est la néphropathie diabétique.

8. Méthode selon la revendication 7, dans laquelle on détermine le taux de transfert d'acide sialique du CMP-N-acétylneuraminate au lactosylcéramide.

9. Méthode selon la revendication 7 ou 8, dans laquelle le donneur et/ou l'accepteur d'acide sialique est marqué de manière détectable.
